(19) Europäisches Patentamt
European Patent Office
Office européen des brevets

(11) Veröffentlichungsnummer: **0 074 595**
**B1**

(12) # EUROPÄISCHE PATENTSCHRIFT

(45) Veröffentlichungstag der Patentschrift: **23.07.86**

(21) Anmeldenummer: **82108192.4**

(22) Anmeldetag: **06.09.82**

(51) Int. Cl.⁴: **C 07 D 239/42,** C 07 D 239/46, C 07 D 239/52, C 07 D 251/16, C 07 D 251/46, A 01 N 47/36

(54) **Substituierte Phenylsulfonylharnstoff- Derivate, Verfahren und neue Zwischenprodukte zu ihrer Herstellung und ihre Verwendung als Herbizide.**

(30) Priorität: **16.09.81 JP 144590/81**

(43) Veröffentlichungstag der Anmeldung: **23.03.83 Patentblatt 83/12**

(45) Bekanntmachung des Hinweises auf die Patenterteilung: **23.07.86 Patentblatt 86/30**

(84) Benannte Vertragsstaaten: **AT BE CH DE FR GB IT LI NL SE**

(56) Entgegenhaltungen:
**EP-A-0 044 211**
**EP-A-0 056 969**
**US-A-4 127 405**

(73) Patentinhaber: **NIHON TOKUSHU NOYAKU SEIZO K.K., No.4, 2-Chome, Nihonbashi Honcho, Chuo-ku Tokyo 103 (JP)**

(72) Erfinder: **Aya, Masahiro, 2-844, Suzuki- cho, Kodaira- shi Tokyo (JP)**
Erfinder: **Saito, Junichi, 3-7- 12, Osawa, Mitaka- shi Tokyo (JP)**
Erfinder: **Yasui, Kazuomi, 7-6- 15, Shakujii- dai Nerima- ku, Tokyo (JP)**
Erfinder: **Shiokawa, Kozo, 210- 6, Shukugawara, Tama- ku, Kawasaki- shi Kanagawa- ken (JP)**
Erfinder: **Morishima, Norihisa, 4-32- 5, Owada- cho, Haichioni- shi Tokyo (JP)**
Erfinder: **Goto, Toshio, 20- 1-304, 5-chome, Seishin, Sagamihara- shi Kanagawa- ken (JP)**

(74) Vertreter: **Schumacher, Günter, Dr., c/o Bayer AG Konzernverwaltung RP Patentabteilung, D-5090 Leverkusen 1 Bayerwerk (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1986

**Beschreibung für die Vertragsstaaten: BE CH DE FR GB IT LI NL und SE**

Die vorliegende Erfindung betrifft bestimmte neue substituierte Phenylsulfonylharnstoff-Derivate, ein Verfahren zu deren Herstellung und deren Verwendung als Herbizide sowie bestimmte substituierte Benzolsulfonylisocyanate als Zwischenprodukte bei deren Herstellung.

In US—A—4 127 405 und US—A— 4 169 719 und in der veröffentlichten japanischen Patentanmeldung Nr. 52—122384 ist offenbart, daß Verbindungen der allgemeinen Formel

$$R_1—SO_2—NH—\overset{\overset{W}{\|}}{C}—NH—R \qquad (V),$$

in der

R 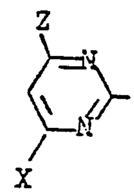 oder 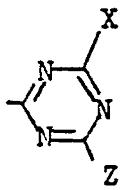 und 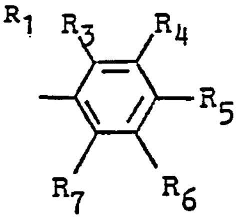 , 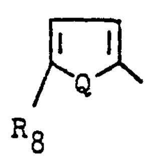 oder 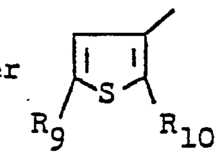

bezeichnen, worin

$R_3$ und $R_6$ unabhängig voneinander ein Wasserstoff-, Fluor-, Brom- oder Iod-Atom, eine Alkyl-Gruppe mit 1 bis 4 Kohlenstoff-Atomen, eine Alkoxy-Gruppe mit 1 bis 4 Kohlenstoff-Atomen, eine Nitro-, Trifluoromethyl- oder Cyano-Gruppe oder eine $CH_3S(O)_n$- oder $CH_3CH_2S(O)_n$-Gruppe (worin n 0, 1 oder 2 ist),

$R_4$ ein Wasserstoff-, Fluor-, Chlor-, Brom-Atom oder eine Methyl-Gruppe,

$R_5$ ein Wasserstoff-, Fluor-, Chlor-, Brom-Atom, eine Methyl-Gruppe oder eine Methoxy-Gruppe,

$R_7$ ein Wasserstoff-, Fluor- oder Brom-Atom, eine Alkyl-Gruppe mit 1 oder 2 Kohlenstoff-Atomen oder eine Alkoxy-Gruppe mit 1 oder 2 Kohlenstoff-Atomen,

$R_8$, $R_9$ und $R_{10}$ unabhängig voneinander ein Wasserstoff-, Chlor- oder Brom-Atom oder eine Methylgruppe,

W und Q unabhängig voneinander ein Sauerstoff- oder Schwefel-Atom,

X ein Wasserstoff-, Chlor- oder Brom-Atom, eine Methyl- oder Ethyl-Gruppe, eine Alkoxy-Gruppe mit 1 bis 3 Kohlenstoff-Atomen, eine Trifluoromethyl-Gruppe oder $CH_3S$— oder $CH_3OCH_2$— und

Z eine Methyl- oder Methoxy-Gruppe

bezeichnen, mit den zusätzlichen Bedingungen, daß

a) wenn $R_5$ kein Wasserstoff-Atom ist, mindestens einer der Substituenten $R_3$, $R_4$, $R_6$ und $R_7$ kein Wasserstoff-Atom ist und mindstens zwei der Substituenten $R_3$, $R_4$, $R_6$ und $R_7$ Wasserstoff-Atome sein müssen,

b) wenn $R_5$ ein Wasserstoff-Atom ist und sämtliche Substituenten $R_3$, $R_4$, $R_6$ und $R_7$ keine Wasserstoff-Atome sind, sämtliche Substituenten $R_3$, $R_4$, $R_6$ und $R_7$ entweder ein Chlor-Atom oder eine Methyl-Gruppe sein müssen, und

c) wenn sowohl $R_3$ als auch $R_7$ Wasserstoff-Atome sind, mindestens einer der Substituenten $R_3$, $R_4$, $R_5$ und $R_6$ ein Wasserstoff-Atom sein muß,

und deren physiologisch unbedenkliche Salze herbizide Wirksamkeit besitzen.

Die herbiziden Eigenschaften dieser Verbindungen sind im Hinblick auf ihren Wirkungsspiegel und ihre Selektivität jedoch nicht immer zufriedenstellend.

In der vorgängigen, jedoch nicht vorveröffentlichten Patentanmeldung EP—A—56 969 werden herbizid wirksame Verbindungen beschrieben, die den erfindungsgemäßen Verbindungen der nachfolgend angegebenen Formel (I) ähnlich sind; jedoch sind die beiden Benzolringe unsubstituiert (d.h. die Indices m und n stehen gleichzeitig für Null).

In der vorgängigen, ebenfalls nicht vorveröffentlichten Patentanmeldung EP—A—44 211 werden herbizid wirksame Verbindungen beschrieben, die gleichfalls Ähnlichkeit haben mit den erfindungsgemäßen Verbindungen der nachfolgend angegebenen Formel (I). Dies gilt für bestimmte Verbindungen der Formel (I), worin X für eine Einfachbindung steht, jedoch nicht für solche Verbindungen, in denen entweder n = 2 ist oder der Rest

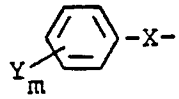

für eine 5-Phenyl- oder eine 2-(4-Nitrophenyl)-Gruppe steht; die letzgenannte Verbindungsgruppe ist neu.

Aus der vorgängigen Patentanmeldung EP—A—21 641 ist es bekannt, daß man die als Zwischenprodukte benötigten Arylsulfonylisocyanate vom Typ der nachfolgend angegebenen Formel (II) in guten Ausbeuten herstellen kann, indem man die entsprechenden Arylsulfonamide in Gegenwart jeweils katalytischer Mengen eines Kohlenwasserstoff-isocyanats und einer tertiären Aminbase phosgeniert.

Gegenstand der vorliegenden Erfindung sind nunmehr, als neue Verbindungen, substituierte Phenylsulfonylharnstoff-Derivate der allgemeinen Formel

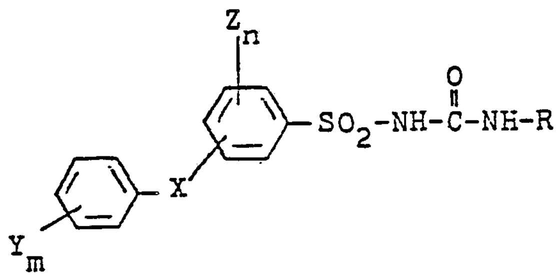

(I)

in der

X für ein Sauerstoff-Atom oder eine Einfachbindung steht,

Y und Z unabhängig voneinander ein Halogen-Atom, eine $C_1$- bis $C_6$-Gruppe, eine $C_1$- bis $C_6$-Alkoxy-Gruppe oder eine Nitro-Gruppe und

R eine Gruppe der allgemeinen Formel

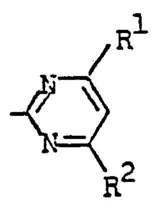

oder

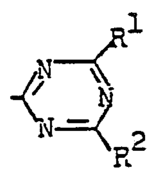

bezeichnen, in der

$R^1$ und $R^2$ unabhängig voneinander für eine $C_1$- bis $C_6$-Alkyl-Gruppe oder eine $C_1$- bis $C_6$-Alkoxy-Gruppe stehen, und

m und n jeweils unabhängig voneinander 0, 1 oder 2 sind, mit der Maßgabe, daß m und n nicht beide gleichzeitig 0 sind, daß Z — wenn n = 2 ist — unterschiedliche Substituenten in Form von Br und $NO_2$ bedeuten kann und daß — wenn X für eine Einfachbindung steht — entweder n = 2 ist oder der Rest

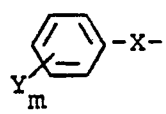

für eine 5-Phenyl- oder eine 2-(4-Nitrophenyl)-Gruppe steht.

Gegenstand der vorliegenden Erfindung ist weiterhin ein Verfahren zur Herstellung einer Verbindung der Formel (I) gemäß der vorliegenden Erfindung, das dadurch gekennzeichnet ist, daß eine Verbindung der allgemeinen Formel

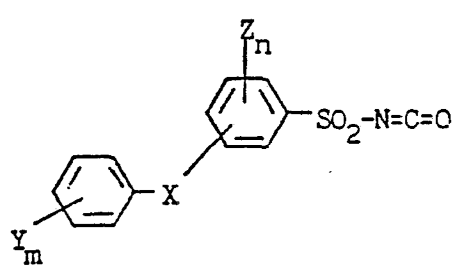

(II),

in der X, Y, Z, m und n die im Vorstehenden angegebenen Bedeutungen haben, mit einer Verbindung der allgemeinen Formel

$$H_2N—R \qquad (III)$$

umgesetzt wird, in der R die vorstehende Bedeutung hat.

Gegenstand der vorliegenden Erfindung sind außerdem, als neue Verbindungen, die substituierten Benzolsulfonylisocyanate der allgemeinen Formel

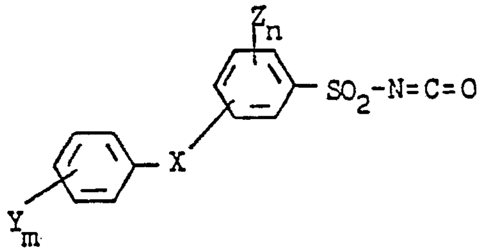

(II)

in der X, Y, Z, m und n die im Vorstehenden angegebenen Bedeutungen haben.

Gegenstand der vorliegenden Erfindung ist außerdem ein Verfahren zur Herstellung einer Verbindung der Formel (II) gemäß der vorliegenden Erfindung, das dadurch gekennzeichnet ist, daß ein substituiertes Benzolsulfonamid der allgemeinen Formel

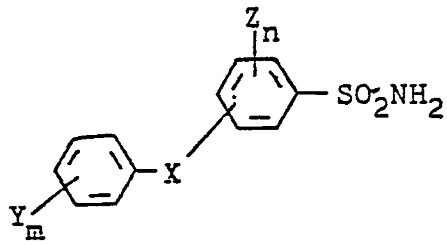

(IV)

in der X, Y, Z, m und n die im Vorstehenden angegebenen Bedeutungen haben, mit Phosgen ($COCl_2$) oder Trichloromethylchlorameisensäureester ($CCl_3OCOCl$) in Gegenwart eines Kohlenwasserstoff-isocyanats umgesetzt wird.

Nunmehr wurde gefunden, daß die substituierten Phenylsulfonylharnstoff-Derivate der Formel (I) eine hervorragende selektive herbizide Wirksamkeit besitzen, die aufgrund der Kenntnis der oben erwähnten Verbindungen gemäß dem Stand der Technik nicht zu erwarten war. Insbesondere zeigen die aktiven Verbindungen gemäß der vorliegenden Erfindung im Vergleich zu konventionellen Herbiziden, die eine beträchtliche Phytotoxizität gegenüber Reispflanzen aufweisen, auch wenn sie ihre herbizide Wirkung bereits bei bemerkenswert niedrigen Dosierungen entfalten, überhaupt keine Phytotoxizität gegen Reispflanzen und eine ausgezeichnete, selektive herbizide Wirkung bei niedrigen Dosierungen. Damit repräsentiert die vorliegende Erfindung einen erheblichen technischen Fortschritt.

Bevorzugte Verbindungen der Formel (I) gemäß der vorliegenden Erfindung sind diejenigen, in denen X für ein Sauerstoff-Atom oder eine Einfachbindung,

Y und Z jeweils unabhängig voneinander für ein Fluor-, Chlor-, Brom- oder Iod-Atom oder eine Methyl-, Ethyl-, Propyl-, Isopropyl-, n- (iso-, sec- oder tert-)Butyl-, Methoxy-, Ethoxy-, Propoxy-, Isopropoxy-, n- (iso-, sec- oder tert-)Butoxy- oder Nitro- Gruppe und

R für eine 2-Pyrimidinyl-Gruppe oder eine 1,3,5-Triazin-2-yl-Gruppe, in denen die 4- und 6-Stellungen des Pyrimidin-Rings oder 1,3,5-Triazin-Rings durch Substituenten ausgewählt aus Methyl, Ethyl, Propyl, Isopropyl, n- (iso-, sec- oder tert-)Butyl, Methoxy, Ethoxy, Propoxy, Isopropoxy und n- (iso-, sec- oder tert-)Butoxy substituiert sind,

stehen und

m und n jeweils unabhängig voneinander 0, 1 oder 2 sind, mit der Maßgabe, daß m und n nicht beide gleichzeitig 0 sind, daß Z — wenn n = 2 ist — unterschiedliche Substituenten in Form von Br und $NO_2$ bedeuten kann und daß — wenn X für eine Einfachbindung steht — entweder n = 2 ist oder der Rest

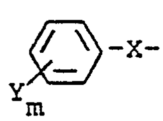

für eine 5-Phenyl- oder eine 2-(4-Nitrophenyl)-Gruppe steht.

Das Verfahren gemäß der vorliegenden Erfindung zur Herstellung der Verbindungen der Formel (I) wird durch die folgende Gleichung beschrieben:

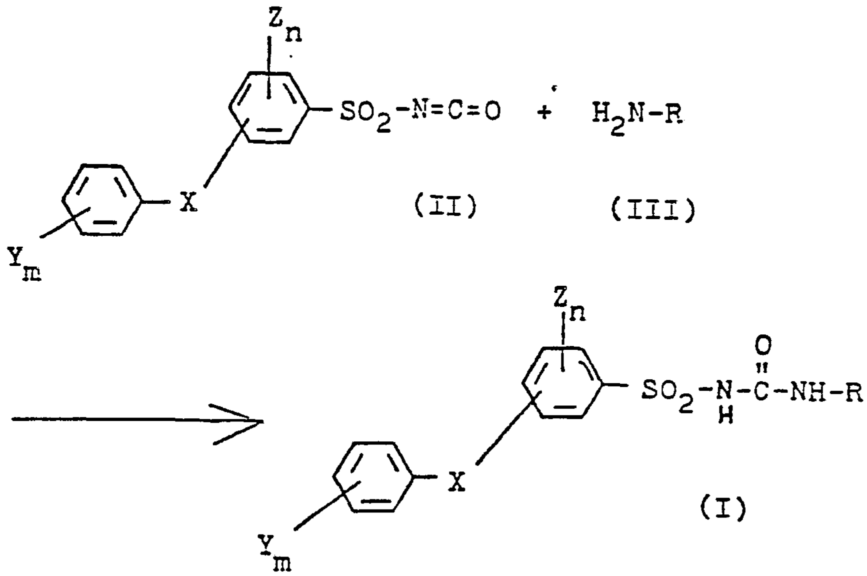

(II) (III)

(I)

in der X, Y, Z, R, m und n die im Vorstehenden angegebenen Bedeutungen haben.

Bevorzugte Ausgangsmaterialien der Formel (II) sind diejenigen, in denen X, Y, Z, m und n die in der Definition der bevorzugten Verbindungen der Formel (I) gemäß der vorliegenden Erfindung angegebenen Bedeutungen haben.

Als Beispiele für die Verbindungen der Formel (II) seien erwähnt:

2-(2-Chlorophenoxy)benzolsulfonylisocyanat,
2-(4-Chlorophenoxy)benzolsulfonylisocyanat,
2-Methoxy-5-phenylbenzolsulfonylisocyanat,
2-(4-Nitrophenyl)benzolsulfonylisocyanat,
2-Bromo-4-nitro-6-phenylbenzolsulfonylisocyanat,
2-(2-Fluorophenoxy)benzolsulfonylisocyanat,
2-Chloro-6-phenoxybenzolsulfonylisocyanat,
2-(o-Tolyloxy)benzolsulfonylisocyanat,
5-Methyl-2-phenoxybenzolsulfonylisocyanat,
2-Ethoxy-5-phenylbenzolsulfonylisocyanat,
2-(4-Isopropylphenoxy)benzolsulfonylisocyanat,
2-(2-Methoxyphenoxy)benzolsulfonylisocyanat und
2-(2,4-Dichlorophenoxy)benzolsulfonylisocyanat.

Bevorzugte Ausgangsmaterialien der Formel (III) sind diejenigen, in denen R die in der Definition der bevorzugten Verbindungen der Formel (I) gemäß der vorliegenden Erfindung angegebene Bedeutung hat.

Als Beispiele für die Verbindungen der Formel (III) seien erwähnt:

2-Amino-4-methoxy-6-methylpyrimidin,
2-Amino-4,6-dimethoxy-1,3,5-triazin,
2-Amino-4-methoxy-6-methyl-1,3,5-triazin,
2-Amino-4,6-dimethoxypyrimidin,
2-Amino-4,6-dimethylpyrimidin,
2-Amino-4,6-dimethyl-1,3,5-triazin,
2-Amino-4,6-diethoxy-1,3,5-triazin,
2-Amino-4,6-dipropylpyrimidin und
2-Amino-4-methyl-6-propoxy-1,3,5-triazin.

Wenn beispielsweise 2-(2-Chlorophenoxy)benzolsulfonylisocyanat und 2-Amino-4-methoxy-6-methylpyrimidin als Ausgangsmaterialien verwendet werden, wird das Verfahren gemäß der vorliegenden Erfindung zur Herstellung der Verbindungen der Formel (I) durch die folgende Gleichung beschrieben:

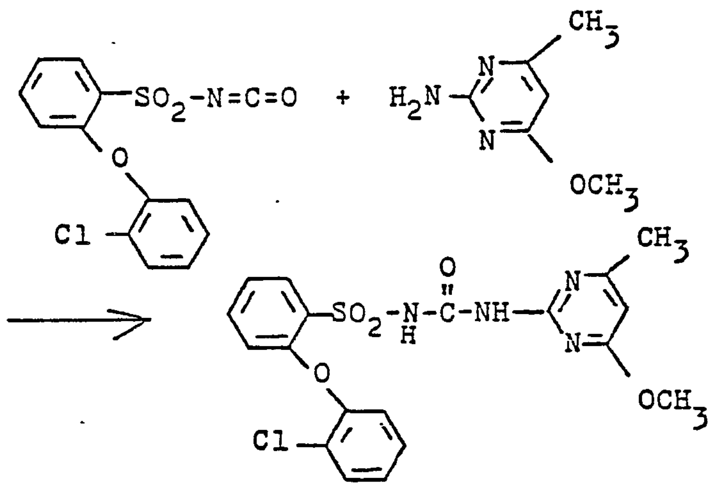

Das Verfahren zur Herstellung von Verbindungen der Formel (I) gemäß der vorliegenden Erfindung wird vorzugsweise unter Verwendung eines Lösungsmittels oder Verdünnungsmittels durchgeführt. Zu diesem Zweck können sämtliche inerten Lösungsmittel oder Verdünnungsmittel verwendet werden. Hierzu zählen insbesondere aliphatische, alicyclische und aromatische, gegebenenfalls chlorierte, Kohlenwasserstoffe (wie Hexan, Cyclohexan, Petrolether, Ligroin, Benzol, Toluol, Xylol, Methylchlorid, Chloroform, Kohlenstofftetrachlorid, Ethylenchlorid, Trichloroethylen und Chlorbenzol), Ether (wie Diethylether, Methylethylether, Di-isopropylether, Dibutylether, Propylenoxid, Dioxan und Tetrahydrofuran), Ketone (wie Aceton, Methylethylketon, Methylisopropylketon und Methylisobutylketon), Nitrile (wie Acetonitril, Propionitril und Acrylnitril), Ester (wie Ethylacetat und Amylacetat), Säureamide (wie Dimethylformamid und Dimethylacetamid), Sulfone und Sulfoxide (wie Dimethylsulfoxid), Sulfolan und Basen (wie Pyridin).

Dieses Verfahren wird zweckmäßigerweise in Gegenwart eines Katalysators wie 1,4-Diazabicyclo-[2,2,2]octan durchgeführt.

Die Reaktionstemperatur kann innerhalb eines beträchtlichen Bereichs variiert werden. Im allgemeinen wird die Reaktion bei einer Temperatur im Bereich von −20°C und dem Siedepunkt der Reaktionsmischung, vorzugsweise zwischen 0°C und 100°C, durchgeführt.

Das Verfahren gemäß der vorliegenden Erfindung wird vorzugsweise unter dem Druck der Umgebung durchgeführt, wenngleich es auch bei erhöhtem oder vermindertem Druck durchgeführt werden kann.

Wie bereits im Vorstehenden erwähnt sind die substituierten Benzolsulfonylisocyanate der vorstehenden Formel (II) neue Verbindungen, die einen weiteren Gegenstand der vorliegenden Erfindung bilden und als Zwischenprodukte auf dem Wege zu den substituierten Phenylsulfonylharnstoff-Derivaten der vorstehenden Formel (I) wertvoll sind, die wie im Vorstehenden beschrieben eine hervorragende selektive herbizide Wirkung besitzen.

Das weitere Verfahren gemäß der vorliegenden Erfindung zur Herstellung der Ausgangsverbindungen der Formel (II) wird durch die folgende Gleichung beschrieben.

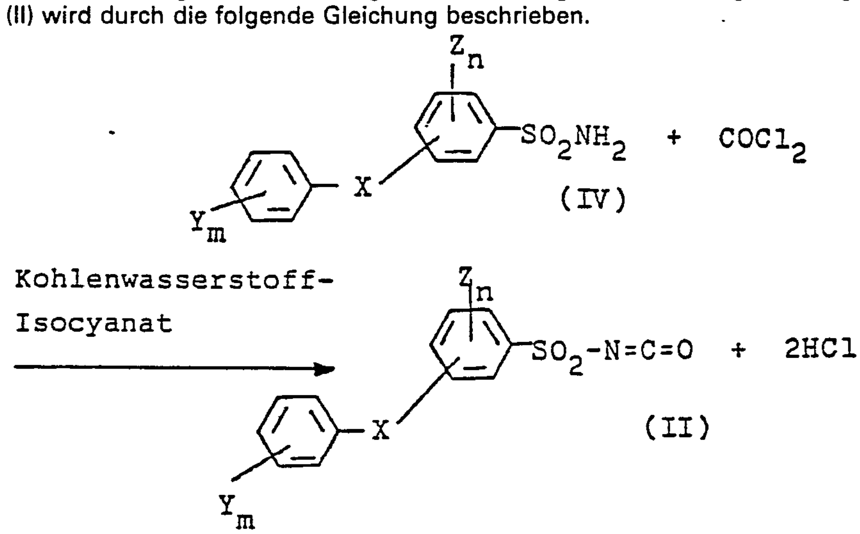


(IV)

(II)

in der X, Y, Z, m und n die im Vorstehenden angegebenen Bedeutungen haben.

In dem vorstehenden Reaktionsschema kann anstelle des Phosgens ($COCl_2$) auch Trichloromethylchlorameisensäureester ($CCl_3OCOCl$) eingesetzt werden.

Spezielle Beispiele für das substituierte Benzolsulfonamid der Formel (IV), das ein Ausgangsstoff für die Herstellung des substituierten Benzolsulfonylisocyanats der Formel (II) ist, sind:

2-(2-Chlorophenoxy)benzolsulfonamid,
2-(4-Chlorophenoxy)benzolsulfonamid,
2-Methoxy-5-phenylbenzolsulfonamid,
2-(4-Nitrophenyl)benzolsulfonamid,
2-Bromo-4-nitro-6-phenylbenzolsulfonamid,
2-(2-Fluorophenoxy)benzolsulfonamid,
2-Chloro-6-phenoxybenzolsulfonamid,
2-(o-Tolyloxy)benzolsulfonamid,
2-Methyl-2-phenoxybenzolsulfonamid,
2-Ethoxy-5-phenylbenzolsulfonamid,
2-(4-Isopropylphenoxy)benzolsulfonamid,
2-(2-Methoxyphenoxy)benzolsulfonamid und
2-(2,4-Dichlorophenoxy)benzolsulfonamid.

Die Reaktion zur Herstellung der Verbindungen der Formel (II) wird in Gegenwart eines Kohlenwasserstoff-Isocyanats durchgeführt, in dem der Kohlenwasserstoff-Rest eine alkyl-Gruppe mit 3 bis 8 Kohlenstoff-Atomen oder eine Cycloalkyl-Gruppe mit 5 bis 8 Kohlenstoff-Atomen sein kann. Spezielle Beispiele für geeignete Kohlenwasserstoff-Isocyanate sind

n-Butylisocyanat,
n-Hexylisocyanat und
Cyclohexylisocyanat.

Wenn beispielsweise 2-(2-Chlorophenoxy)benzolsulfonamid, Trichloromethylchlorameisensäureester und n-Butylisocyanat als Reaktionsteilnehmer eingesetzt werden, wird das Verfahren gemäß der vorliegenden Erfindung zur Herstellung der Verbindungen der Formel (II) durch die folgende Gleichung beschrieben:

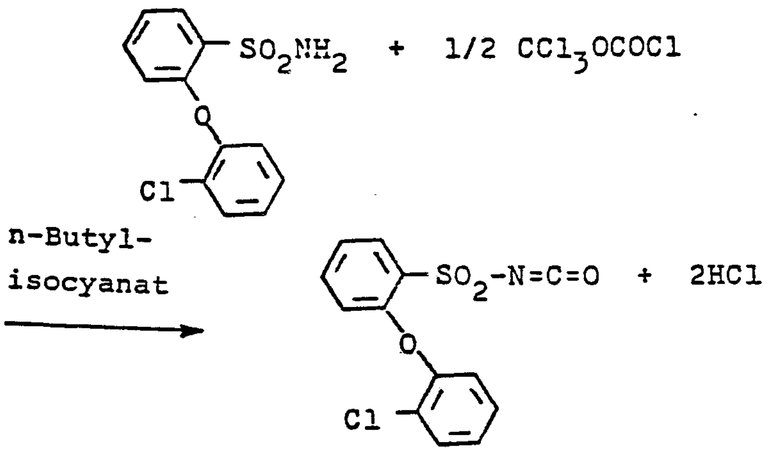


Das Kohlenwasserstoff-Isocyanat, das ein wesentlicher Reaktionsteilnehmer für die Erzeugung der Verbindungen der Formel (II) ist, kann am Ende der Reaktion zurückgewonnen werden, und infolgedessen ist das Verfahren für die Industrie vorteilhaft.

Das Verfahren zur Herstellung der Verbindungen der Formel (II) kann in jedem der inerten organischen Lösungsmittel oder Verdünnungsmittel durchgeführt werden, die weiter oben als Verdünnungsmittel bei der Herstellung der Verbindungen der Formel (I) genannt wurden.

Weiterhin wird das Verfahren zur Herstellung der Verbindungen der Formel (II) zweckmäßigerweise in Gegenwart eines Katalysators wie tertiärer Amine (beispielsweise Triethylamin, Dimethylcyclohexylamin und 1,4-Diazabicyclo[2,2,2]octan) durchgeführt.

Die Reaktionstemperatur kann innerhalb eines beträchtlichen Bereichs variiert werden. Im allgemeinen wird die Reaktion bei einer Temperatur im Bereich von −20°C und dem Siedepunkt der Reaktionsmischung, vorzugsweise zwischen 0°C und 100°C, durchgeführt.

Das Verfahren gemäß der vorliegenden Erfindung zur Herstellung der Verbindungen der Formel (II) wird vorzugsweise unter dem Druck der Umgebung durchgeführt, wenngleich es auch bei erhöhtem oder vermindertem Druck durchgeführt werden kann.

Die erfindungsgemäßen Wirkstoffe können als Mittel zur Bekämpfung von Unkräutern verwendet werden.

Unter "Unkräutern" im weitesten Sinne sind solche Pflanzen zu verstehen, die an Stellen wachsen, wo sie nicht erwünscht sind.

Die aktiven Verbindungen gemäß der vorliegenden Erfindung sind hervorragend hinsichtlich ihrer Sicherheit, zeigen eine überlegene herbizide Wirkung und besitzen ein breites herbizides Spektrum.

Die aktiven Verbindungen gemäß der vorliegenden Erfindung können beispielsweise zur Bekämpfung der folgenden Pflanzen verwendet werden:

Dikotyledonen-Unkräuter der Gattungen
Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbanda, Ambrosia, Cirsium, Carduus, Sonchus, Rorippa, Rotala, Lindernia, Lamium, Veronica, Abutilon, Emex, Datura, Viola, Galeopsis, Papaver, Centaurea und Solanum; und

Monokotyledonen-Unkräuter der Gattungen
Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Die aktiven Verbindungen gemäß der vorliegenden Erfindung können beispielsweise als selektive Herbizide in den folgenden Kulturen verwendet werden:

Dikotyledonen-Kulturen der Gattungen
Gossypium, Glycine, Beta, Daucus, Phaseolus, Pisum, Solanum, Linum, Ipomoea, Vicia, Nicotiana, Lycopersilicon, Arachis, Brassica, Lactuca, Cucumis und Cucurbita; und

Monokotyledonen-Kulturen der Gattungen
Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleich Weise.

In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in einjährigen Kulturen verwendet werden.

Die aktiven Verbindung gemäß der vorliegenden Erfindung zeigen eine überlegene selektive Bekämpfungswirkung, insbesondere wenn sie als Mittel zur Bodenbehandlung von der Keimung oder als Mittel zur Behandlung der Stengel und Blätter von Unkräutern und des Bodens auf Reisfeldern eingesetzt werden. Beispielsweise entfalten sie herbizide Wirkung gegen solche Unkräuter auf Reisfeldern wie die nachstehend aufgeführten, ohne daß sie irgend eine schädliche Wirkung auf die Reispflanzen ausüben:

Die Dikotyledonen-Unkräuter
Rotala indica Koehne,
Lindernia procumbens Philcox,
Ludwiga prostrata Roxburgh,
Potamogeton distinctus A. Bennett,
Elatine triandra Schkuhr
sowie
die Monokotyledonen-Unkräuter
Echinochloa crus-galli Beauvois var.,
Monochoria vaginalis Presl,
Eleocharis acicularis L.,
Eleocharis kuroguwai Ohwi,
Cyperus difformis L.,
Cyperus serotinus Rottboell,
Sagittaria pygmaea Miquel,
Alisma canaliculatum A. Braun et Bouché und
Scirpus juncoides Roxburgh var.

Die Wirkstoffe können in übliche Präparate wie Lösungen, Emulsionen, Suspensionen, Pulver, Stäubemittel, Schäume, Pasten, lösliche Pulver, Granulat, Aerosole, Suspensions-Emulsions-Konzentrate, mit dem Wirkstoff imprägnierte natürliche und synthetische Materialien, sehr feine Kapseln in polymeren Substanzen, sowie ULV-Kaltvernebelungs- und Warmvernebelungspräparate umgewandelt werden.

Diese Präparate können in bekannter Weise hergestellt werden, beispielsweise durch Vermischen der Wirkstoffe mit Streckmitteln, d.h. flüssigen oder festen Verdünnungsmitteln oder Trägern, gegebenenfalls unter Verwendung von oberflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger, insbesondere als Lösungsmittel, eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen oder organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate und Arylsulfonate sowie Hydrolyseprodukte des Albumins verwendet werden. Zu den Dispergiermitteln zählen beispeilsweise Ligninsulfit-Ablaugen und Methylcellulose.

Haftmittel wie Carboxymethylcellulose und natürliche und synthetische Polymerisate in Form von Pulver, Granulat oder Latices, z.B. Gummiarabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe und Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,001 bis 100 Gewichts-%, vorzugsweise von 0,05 bis 95 Gewichts-% des Wirkstoffes.

Die Wirkstoffe gemäß der vorliegenden Erfindung können in Präparaten oder verschiedenen Anwendungsformen als solche oder in Verbindung mit anderen aktiven Verbindungen wie Fungiziden, Bakteriziden, Insektiziden, Nematiziden, vogelabweisenden Mitteln, Wachstumsfaktoren, Pflanzennährstoffen sowie Mitteln zur Verbesserung der Bodenstruktur eingesetzt werden.

Insbesondere kann durch Zusatz geeigneter Mengen anderer Wirkstoffe zu den aktiven Verbindungen gemäß der vorliegenden Erfindung ein breiteres herbizides Spektrum und eine genauere Bekämpfungswirkung erreicht werden, und aufgrund einer solchen Vermischung kann auch eine synergistische Wirkung erreicht werden. Beispiele für solche anderen Wirkstoffe sind:

2-Chloro-2′,6′-diethyl-N-(butoxymethyl)-acetanilid,
N-(O,O-Dipropyl-diethyl-phosphorylacetyl)-2-methylpiperidin,
S-(4-Chlorobenzyl)-N,N-diethylthiolcarbamat,
S-Ethyl-N,N-hexamethylenthiolcarbamat,
O-Methyl-O-(2-nitro-p-tolyl)-N-isopropylphosphoroamidthioat,
O-Ethyl-O-(2-nitro-5-methylphenyl)-N-sec-butylphosphoroamidthioat,
3,4-Dimethyl-2,6-dinitro-N-1-ethylpropylanilid,
α,α,α-Trifluoro-2,6-dinitro-N,N-dipropyl-p-toluidin,
2-Chloro-2′,6′-diethyl-N-(n-propoxyethyl)-acetanilid,
4,5-Dichloro-1,3-thiazol-2-yloxyessigsäure-N-isopropyl-N-ethoxyethoxyamid,
5-Ethyl-1,3,4-thiadiazol-2-yloxyessigsäure-1′,2′,3′,4′-tetrahydrochinolid,
Benzothiazol-2-yloxyessigsäure-N,N-diallylamid,
Benzoxazol-2-yloxyessigsäure-N-sec-butyl-N-methylamid,
Benzoxazol-2-yloxyessigsäure-N-cyclohexyl-N-methylamid,
Benzothiazol-2-yloxyessigsäure-N-methyl-N-(1-methylpropargyl)amid,
Benzoxazol-2-yloxyessigsäure-N-benzyl-N-propargylamid,
Benzothiazol-2-yloxyessigsäure-2′-ethylpiperidid,
Benzothiazol-2-yloxyessigsäure-2′,4′-dimethylpiperidid,
Benzoxazol-2-yloxyessigsäure-2′,4′,6′-trimethylpiperidid,
Benzoxazol-2-yloxyessigsäure-hexamethylenimid,
Benzothiazol-2-yloxyessigsäure-perhydroindolid,
Benzoxazol-2-yloxyessigsäure-perhydroindolid,
Benzothiazol-2-yloxyessigsäure-1′,2′,3′,4′-tetrahydrochinolid,
Benzoxazol-2-yloxyessigsäure-2′-methyl-1′,2′,3′,4′-tetrahydrochinolid,
Benzoxazol-2-yloxyessigsäure-N-methylanilid,
Benzothiazol-2-yloxyessigsäure-N-methylanilid,
Benzoxazol-2-yloxyessigsäure-N-ethylanilid,
Benzoxazol-2-yloxyessigsäure-N-propylanilid,
Benzoxazol-2-yloxyessigsäure-N-isopropylanilid,
Benzothiazol-2-yloxyessigsäure-N-methyl-N-2′-methoxyanilid,

Benzoxazol-2-yloxyessigsäure-N-methyl-N-2'-methoxyanilid,
Benzoxazol-2-yloxyessigsäure-N-methyl-N-2'-trifluoromethylanilid,
Benzothiazol-2-yloxyessigsäure-N-methyl-N-2'-chloroanilid,
Benzoxazol-2-yloxyessigsäure-N-methyl-N-2'-chloroanilid,
Benzothiazol-2-yloxyessigsäure-N-methyl-N-2'-fluoroanilid,
Benzoxazol-2-yloxyessigsäure-N-methyl-N-2'-fluoroanilid,
Benzothiazol-2-yloxyessigsäure-N-methyl-N-3'-methylanilid,
Benzoxazol-2-yloxyessigsäure-N-methyl-N-3'-methylanilid,
Benzothiazol-2-yloxyessigsäure-N-methyl-N-3'-methoxyanilid,
Benzoxazol-2-yloxyessigsäure-N-methyl-N-3'-methoxyanilid,
Benzothiazol-2-yloxyessigsäure-N-methyl-N-3'-isopropylanilid,
Benzoxazol-2-yloxyessigsäure-N-methyl-N-3'-isopropoxyanilid,
Benzothiazol-2-yloxyessigsäure-N-methyl-N-3'-trifluoromethylanilid,
Benzoxazol-2-yloxyessigsäure-N-methyl-N-3'-trifluoromethylanilid,
Benzothiazol-2-yloxyessigsäure-N-methyl-N-3'-chloroanilid,
Benzoxazol-2-yloxyessigsäure-N-methyl-N-3'-chloroanilid,
Benzothiazol-2-yloxyessigsäure-N-methyl-N-3'-fluoroanilid,
Benzoxazol-2-yloxyessigsäure-N-methyl-N-3'-fluoroanilid,
Benzothiazol-2-yloxyessigsäure-N-methyl-N-3'-bromoanilid,
Benzoxazol-2-yloxyessigsäure-N-methyl-N-3'-bromoanilid,
Benzoxazol-2-yloxyessigsäure-N-methyl-N-4'-methylanilid,
Benzoxazol-2-yloxyessigsäure-N-methyl-N-4'-methoxyanilid,
Benzoxazol-2-yloxyessigsäure-N-methyl-N-4'-fluoroanilid,
Benzoxazol-2-yloxyessigsäure-N-methyl-N-2',3'-dimethylanilid,
Benzoxazol-2-yloxyessigsäure-N-methyl-N-2',3'-dichloroanilid,
Benzoxazol-2-yloxyessigsäure-N-methyl-N-4'-chloro-2'-methylanilid,
Benzothiazol-2-yloxyessigsäure-N-methyl-N-2',5'-dichloroanilid,
Benzoxazol-2-yloxyessigsäure-N-methyl-N-2',5'-dichloroanilid,
Benzothiazol-2-yloxyessigsäure-N-methyl-N-3',5'-dimethylanilid,
Benzoxazol-2-yloxyessigsäure-N-methyl-N-3',5'-dimethylanilid,
Benzoxazol-2-yloxyessigsäure-N-methyl-N-3',5'-ditrifluoromethyl-anilid,
Benzoxazol-2-yloxyessigsäure-N-methyl-N-5'-indanylamid,
Benzothiazol-2-yloxyessigsäure-N-methyl-N-3'-ethylanilid,
Benzoxazol-2-yloxyessigsäure-N-methyl-N-3'-ethylanilid und
Benzothiazol-2-yloxyessigsäure-N-isopropylanilid.

Die Wirkstoffe können als soche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Bewässern, Sprühen, Zerstäuben, Streuen oder Stäuben.

Die Wirkstoffe gemäß der vorliegenden Erfindung können vor oder nach dem Auflaufen der Pflanzen zur Anwendung gelangen, werden jedoch vorzugsweise vor dem Auflaufen der Pflanzen, d.h. mittels des Verfahrens der Vorauflaufbehandlung, angewandt. Sie können auch vor der Einsaat in den Boden eingearbeitet werden.

Es ist auch möglich, die aktiven Verbindungen mittels des ULV-Verfahrens (ultra-low-volume method) zur Anwendung zu bringen, wodurch es möglich wird, die Verbindungen in einer Konzentration bis zu 100 Gew.-% einzusetzen.

Im praktischen Einsatz betragt der Gehalt der Wirkstoffe in der verschiedenen Anwendungsformen oder gebrauchsfertigen Präparaten im allgemeinen von 0,01 bis 95 Gewichts-%, vorzugsweise von 0,05 bis 60 Gewichts-%.

Die flächenbezogenen Aufwandsmengen der aktiven Verbindung betragen im allgemeinen 0,1 bis 3 kg/ha, vorzugsweise 0,2 bis 1 kg/ha. In besonderen Fällen ist es jedoch möglich oder manchmal sogar notwendig, auch eine höhere oder niedrigere Menge außerhalb des genannten Bereichs aufzuwenden.

Die vorliegende Erfindung umfaßt weiter ein herbizides Mittel, das als Wirkstoff eine Verbindung gemäß der vorliegenden Erfindung im Gemisch mit einem festen oder aus einem verflüssigtem Gas bestehenden Verdünnungsmittel oder Träger oder im Gemisch mit einem ein oberflächenaktives Mittel enthaltenden flüssigen Verdünnungsmittel oder Träger enthält.

Die vorliegende Erfindung umfaßt weiter ein Verfahren zur Unkrautbekämpfung, das dadurch gekennzeichnet ist, daß eine Verbindung gemäß der vorliegenden Erfindung allein oder in Form eines Präparats, das als Wirkstoff eine Verbindung gemäß der vorliegenden Erfindung im Gemisch mit einem Verdünnungsmittel oder Träger enthält, auf die Unkräuter oder deren Lebensraum aufgebracht wird.

Die vorliegende Erfindung ist ferner auf Nutzpflanzen gerichtet, die dadurch gegen Schäden durch Unkräuter geschützt sind, daß sie auf Flächen angebaut werden, auf die unmittelbar vor und/oder während der Zeit des Anbauens eine Verbindung gemäß der vorliegenden Erfindung allein oder im Gemisch mit einem Verdünnungsmittel oder Träger aufgebracht wurde.

Es wird ersichtlich werden, daß die üblichen Methoden der Erzeugung geernteter Nutzpflanzen durch die vorliegende Erfindung verbessert werden können.

Zusammensetzungen gemäß der vorliegenden Erfindung werden in den folgenden Beispielen (i) bis (vi) erläutert. Hierin werden die Verbindungen der vorliegenden Erfindung durch die (in Klammer angegebene) Zahl des betreffenden Herstellungsbeispiels bezeichnet.

Beispiel i

15 Gew.-Teile der Verbindung (1), 80 Gew.-Teile eines 1:5-Gemisches aus pulvriger Diatomeenerde und Tonpulver, 2 Gew.-Teile Natriumalkylbenzolsulfonat und 3 Gew.-Teile Natriumalkylnaphthalinsulfonat/Formaldehyd-Kondensat wurden vermahlen und zu einem benetzbaren Pulver vermischt. Vor dem Spritzen wurde es mit Wasser verdünnt.

Beispiel ii

10 Gew.-Teile der Verbindung (2), 75 Gew.-Teile Dimethylformamid, 8 Gew.-Teile Polyoxyethylen-alkylphenylether und 7 Gew.-Teile Calciumalkylbenzolsulfonat wurden unter Rühren miteinander vermischt, wodurch eine Emulsion erhalten wurde. Vor dem Spritzen wurde sie mit Wasser verdünnt.

Beispiel iii

2 Gew.-Teile der Verbindung (3) und 98 Gew.-Teile Tonpulver wurden vermahlen und gemischt, wodurch ein Stäubemittel erhalten wurde.

Beispiel iv

1,5 Gew.-Teile der Verbindung (4), 0,5 Gew.-Teile Isopropylhydrogenphosphat (PAP) und 98 Gew.-Teile Tonpulver wurden vermahlen und gemischt, wodurch ein Stäubemittel erhalten wurde.

Beispiel v

10 Gew.-Teile der Verbindung (5), 30 Gew.-Teile Bentonit (Montmorillonit), 58 Gew.-Teile Talkum und 2 Gew.-Teile Ligninsulfonat-Salz wurden miteinander vermischt, und nach Zusatz von 25 Gew.-Teilen Wasser wurde das Gemisch gut durchgemischt. Die erhaltene Mischung wurde mittels eines Extruder-Granulators zu einem Granulat mit einer Korngröße von 0,43 bis 2,0 mm (10 bis 40 mesh) verarbeitet und bei 40°C bis 50°C getrocknet, wodurch ein Granulat erhalten wurde. Dieses wurde durch Streuen aufgebracht.

Beispiel vi

95 Gew.-Teile Tonmineral-Teilchen mit einer Teilchengrößen-Verteilung im Bereich von 0,2 bis 2 mm wurden in einen Drehmischer gegeben, mit 5 Gew.-Teilen der Verbindung (6) durch Aufspritzen einer Lösung der Verbindung (6) in einem organischen Lösungsmittel gleichmäßig benetzt und dann bei 40°C bis 50°C getrocknet, wodurch ein Granulat erhalten wurde. Dieses wurde durch Streuen aufgebracht.

Die herbizide Wirkung der Verbindungen gemäß der vorliegenden Erfindung wird durch das folgende Biotest-Beispiel aufgezeigt.

In diesem Beispiel werden die Verbindungen gemäß der vorliegenden Erfindung jeweils durch die (in Klammer angegebene) Zahl des betreffenden Herstellungsbeispiels bezeichnet.

Die bekannte Vergleichsverbindung wird durch die nachstehenden Formel beschrieben:

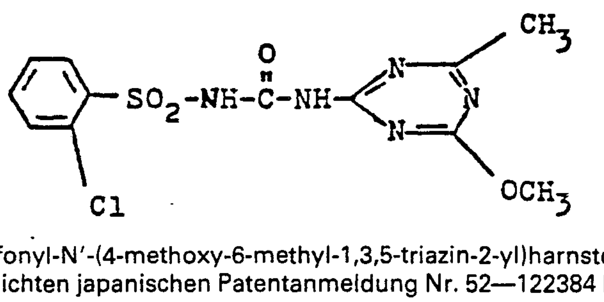

(V-1)

N-2-Chlorophenylsulfonyl-N'-(4-methoxy-6-methyl-1,3,5-triazin-2-yl)harnstoff
(eine in der veröffentlichten japanischen Patentanmeldung Nr. 52—122384 beschriebene Verbindung).

Beispiel A

Prüfung der Wirkung gegen im Wasser wachsende Reisfeld-Unkräuter durch Behandlung des Bodens sowie der Stengel und Blätter unter Bewässerungs-Bedingungen (Topf-Test):

Lösungsmittel: 5 Gewichtsteile Aceton;
Emulgator: 1 Gewichtsteil Benzyloxypolyglycolether.

Zur Herstellung eines geeigneten Wirkstoff-Präparats wurde 1 Gew.-Teil der aktiven Verbindung mit der vorbezeichneten Menge Lösungsmittel vermischt; die angegebene Menge Emulgator wurde zugesetzt, und das Konzentrat wurde mit Wasser auf die gewünschte Konzentration verdünnt.

Reis-Kulturboden wurde in Wagner-Töpfe (2 $dm^2$) gefüllt, und zwei Reis-Setzlinge (Varietät: Kinmaze) im 2- bis 3-Blattstadium (Pflanzenhöhe etwa 10 cm) wurden in jeden Topf umgepflanzt. Samen von Echinochloa crus-galli Beauvois var., Cyperus iria L., Monochoria vaginalis Presl, Scirpus juncoides Roxburgh var. und breitblättrigen Unkräutern, kleine Stücke von Eleocharis acicularis L. und Knollen von Cyperus serotinus Rottboell und Sagittaria pygmaea Miquel wurden eingesetzt, und die Töpfe wurden in feuchtem Zustand gehalten. Als Echinochloa crus-galli Beauvois var. etwa bis zum 2-Blatt-Stadium gewachsen war (etwa 7 bis 9 Tage nach der Einsaat), wurden die Töpfe mit Wasser bis zu einer Tiefe von etwa 6 cm gefüllt, und eine vorher festgelegte Menge des Wirkstoffs wurde in Form einer Emulsion mittels einer Pipette dem Wasser zugegeben. Nach der Behandlung wurde das Wasser im Laufe von zwei Tagen mit einer Geschwindigkeit von 2 bis 3 cm pro Tag aus den Töpfen heraussickern gelassen. Danach wurde die Wassertiefe in den Töpfen bei etwa 3 cm gehalten. Vier Wochen nach der chemischen Behandlung wurden die herbizide Wirkung und der Grad der Phytotoxizität mit Hilfe einer Skala von 0 bis 10 nach dem folgenden Maßstab bewertet.

Die Wirkungen wurden als Unkrautvernichtungsrate mit Hilfe der nachstehenden Skala von 0 bis 10 im Vergleich zu unbehandelten Kontrollpflanzen bewertet:

| Bewertung | Unkrautvernichtungsrate (bezogen auf die unbehandelten Kontrollpflanzen) |
|---|---|
| 10 | 100% (verdorrt) |
| 9 | mindestens 90%, jedoch weniger als 100% |
| 8 | mindestens 80%, jedoch weniger als 90% |
| 7 | mindestens 70%, jedoch weniger als 80% |
| 6 | mindestens 60%, jedoch weniger als 70% |
| 5 | mindestens 50%, jedoch weniger als 60% |
| 4 | mindestens 40%, jedoch weniger als 50% |
| 3 | mindestens 30%, jedoch weniger als 40% |
| 2 | mindestens 20%, jedoch weniger als 30% |
| 1 | mindestens 10%, jedoch weniger als 20% |
| 0 | weniger als 10% (unwirksam) |

Die Phytotoxität wurde mit Hilfe der nachstehenden Skala von 0 bis 10 im Vergleich zu unbehandelten Kontrollpflanzen bewertet:

| Bewertung | Phytotoxizitätsrate (bezogen auf die unbehandelten Kontrollpflanzen) |
|---|---|
| 10 | mindestens 90% (Ausrottung) |
| 9 | mindestens 80%, jedoch weniger als 90% |
| 8 | mindestens 70%, jedoch weniger als 80% |
| 7 | mindestens 60%, jedoch weniger als 70% |
| 6 | mindestens 50%, jedoch weniger als 60% |
| 5 | mindestens 40%, jedoch weniger als 50% |
| 4 | mindestens 30%, jedoch weniger als 40% |
| 3 | mindestens 20%, jedoch weniger als 30% |
| 2 | mindestens 10%, jedoch weniger als 20% |
| 1 | mehr als 10%, jedoch weniger als 10% |
| 0 | 0% (keine Phytotoxizität) |

Die Testergebnisse sind in Tabelle 1 aufgeführt.

TABELLE 1

| Verbindung* | Wirkstoff-Menge kg/ha | Herbizide Wirkung Unkräuter A | B | C | D | E | F | G | H | Phytotoxität gegen Reis |
|---|---|---|---|---|---|---|---|---|---|---|
| (1) | 0,2 | 10 | 10 | 10 | 10 | 10 | 10 | 10 | 10 | 0 |
| (2) | 0,2 | 10 | 10 | 10 | 10 | 10 | 10 | 10 | 10 | 0 |
| (3) | 0,2 | 10 | 10 | 10 | 10 | 10 | 10 | 10 | 10 | 0 |
| (5) | 0,2 | 10 | 10 | 10 | 10 | 10 | 10 | 10 | 10 | 0 |
| (16) | 0,2 | 10 | 10 | 10 | 10 | 10 | 10 | 10 | 10 | 0 |
| (17) | 0,2 | 10 | 10 | 10 | 10 | 10 | 10 | 10 | 10 | 0 |
| (18) | 0,2 | 10 | 10 | 10 | 10 | 10 | 10 | 10 | 10 | 0 |
| (20) | 0,2 | 10 | 10 | 10 | 10 | 10 | 10 | 10 | 10 | 0 |
| (V—1) | 0,2 | 10 | 9 | 10 | 10 | 10 | 10 | 5 | 10 | 9 |

*Anmerkung:*

Die Symbole A bis H bezeichnen die nachstehenden Unkräuter:

A: Echinochloa crus-galli Beauv.var.
B: Eleocharis acicularis L.
C: Cyperus iria L.
D: Scirpus juncoides Roxburgh var.
E: Monochoria vaginalis Presl
F: Breitblättrige Unkräuter (darunter Lindernia procumbens Philcox, Rotala indica Koehne, Elatine triandra Schkuhr)
G: Cyperus serotinus Rottboell
H: Sagittaria pygmaea Miq.

* Die Strukturformeln der unit Ziffern bezeichneten Verbindungen sind in den nachstehenden Tabellen angegeben.

Auch für die Verbindungen (4), (6) bis (15), (19), (21), (22), (23) und (24) wurde aufgrund von ähnlichen Tests wie im vorstehenden Beispiel A bestätigt, daß sie eine ausgezeichnete selektive herbizide Wirkung und dabei keinerlei Phytotoxizität gegenüber Reispflanzen aufweisen.

Die folgenden Beispiele 1 bis 24 beschreiben die Herstellung von Verbindungen der Formel (II), und die Beispiele 25 bis 37 erläutern die Herstellung der Ausgangsverbindungen der Formel (II).

*Herstellungsbeispiele*

Beispiel 1

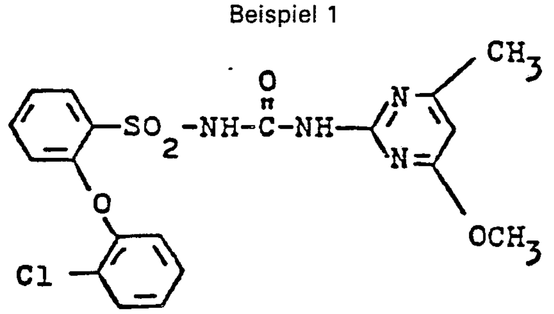

(1)

3,1 g 2-(2-Chlorophenoxy)benzolsulfonylisocyanat wurden in 13 ml Xylol gelöst. Diese Lösung wurde tropfenweise unter Rühren zu einer Suspension von 1,39 g 2-Amino-4-methoxy-6-methylpyrimidin in 13 ml Acetonitril hinzugegeben. Nach beendeter Zugabe wurde die Lösung 1 h zum Rückfluß erhitzt und dann über Nacht bei Raumtemperatur stehen gelassen. Die entstandenen Kristalle wurden abfiltriert, wodurch

4,3 g des gewünschten Produkts, N-2-(2-Chlorophenoxy)-phenylsulfonyl-N'-(4-methoxy-6-methyl-2-pyrimidyl)harnstoff erhalten wurden; Schmp. 216—220°C.

Die in der nachstehenden Tabelle 2 aufgeführten Verbindungen werden wie in Beispiel 1 beschrieben hergestellt, wobei die entsprechenden geeigneten Ausgangsstoffe verwendet werden.

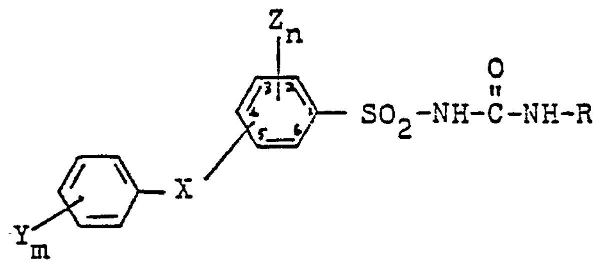

(I)

TABELLE 2

| Beispiel Nr. | 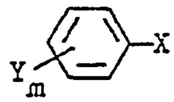 | n | Z | R | Schmelzpunkt (°C) |
|---|---|---|---|---|---|
| 2 | 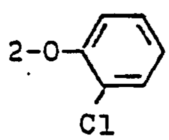 | 0 | - | 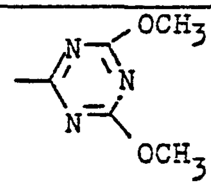 | 201-204 |
| 3 | 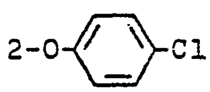 | 0 | - | 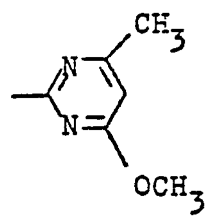 | 213-215 |
| 4 | 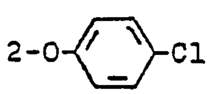 | 0 | - | 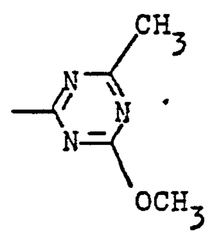 | 157-160 |

TABELLE 2 (Continued)

| Beispiel Nr. | 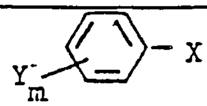 | n | Z | R | Schmelzpunkt (°C) |
|---|---|---|---|---|---|
| 5 | 2-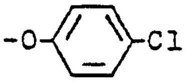 | 0 | - | 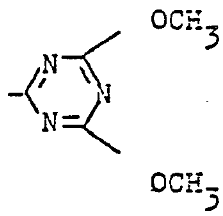 | 155-158 |
| 6 | 5-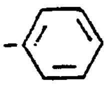 | 1 | 2-$OCH_3$ | 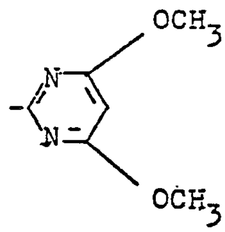 | 217-220 |
| 7 | 5-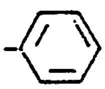 | 1 | 2-$OCH_3$ | 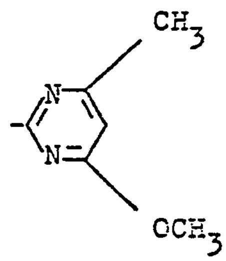 | 203-205 |
| 8 | 5-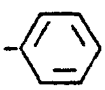 | 1 | 2-$OCH_3$ | 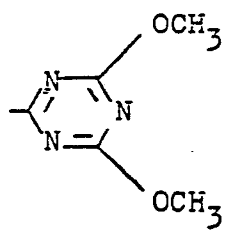 | 151-152 |
| 9 | 2-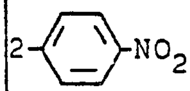 | 0 | - | 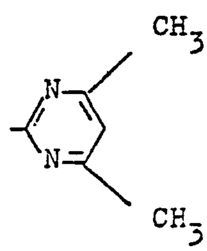 | 203-204 |

TABELLE 2 (Continued)

| Bei-spiel Nr. | 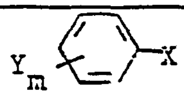 | n | Z | R | Schmelzpunkt (°C) |
|---|---|---|---|---|---|
| 10 | 2-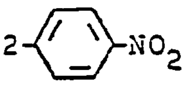 | 0 | - | 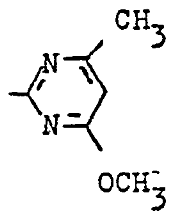 | 207-210 |
| 11 | 2-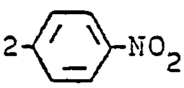 | 0 | - | 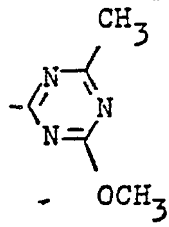 | 181-185 |
| 12 | 2-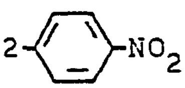 | 0 | - | 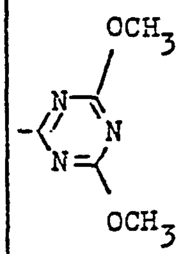 | 175-177 |
| 13 | 2-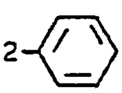 | 2 | 4-$NO_2$ 6-Br | 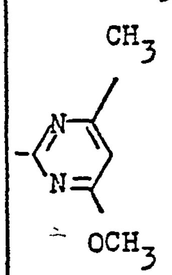 | 146-149 |
| 14 | 2-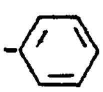 | 2 | 4-$NO_2$ 6-Br | 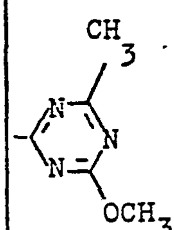 | 137-140 |
| 15 | 2-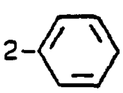 | 2 | 4-$NO_2$ 6-Br | 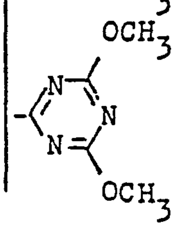 | 168-170 |

Die in der nachstehenden Tabelle 3 aufgeführten Verbindungen wurden nach der in Beispiel 1 beschriebenen Verfahrensweise unter Verwendung der angegebenen Ausgangsstoffe hergestellt.

TABELLE 3

| Beispiel Nr. | Ausgangsstoff | Ausgangsstoff | Produkt |
|---|---|---|---|
| 16 | 2-(2-Chlorophenoxy)benzol-sulfonylisocyanat | 2-Amino-4-methoxy-6-methyl-1,3,5-triazin | N-[2-(2-Chlorophenoxy)phenylsulfonyl]-N'-4-methoxy-6-methyl-1,3,5-triazin-2-yl)harnstoff |
| 17 | 2-(2-Chlorophenoxy)benzol-sulfonylisocyanat | 2-Amino-4,6-dimethoxy-pyrimidin | N-[2-(2-Chlorophenoxy)phenyl-sulfonyl]-N'-4,6-dimethoxy-2-pyrimidyl)harnstoff |
| 18 | 2-(2-Fluorophenoxy)benzol-sulfonylisocyanat | 2-Amino-4,6-dimethoxy-1,3,5-triazin | N-[2-(2-Fluorophenoxy)phenyl-sulfonyl]-N'-4,6-dimethoxy-1,3,5-triazin-2-yl)harnstoff |
| 19 | 2-(2-Fluorophenoxy)benzol-sulfonylisocyanat | 2-Amino-4-methoxy-6-methyl-1,3,5-triazin | N-[2-(2-Fluorophenoxy)phenyl-sulfonyl]-N'-4-methoxy-6-methyl-1,3,5-triazin-2-yl)harnstoff |
| 20 | 2-Chloro-6-phenoxybenzol-sulfonylisocyanat | 2-Amino-4-methoxy-6-methyl-1,3,5-triazin | N-2-Chloro-6-phenoxyphenylsulfonyl-N'-(4-methoxy-6-methyl-1,3,5-triazin-2-yl)harnstoff |
| 21 | 2-Chloro-6-phenoxybenzol-sulfonylisocyanat | 2-Amino-4,6-dimethyl-pyrimidin | N-2-Chloro-6-phenoxyphenylsulfonyl-N'-(4,6-dimethyl-2-pyrimidyl)-harnstoff |
| 22 | 5-Methyl-2-phenoxybenzol sulfonylisocyanat | 2-Amino-4,6-dimethoxy-1,3,5-triazin | N-(5-Methyl-2-phenoxyphenyl-sulfonyl)-N'-(4,6-dimethoxy-1,3,5-triazin-2-yl)harnstoff |
| 23 | 2-(2-Methoxyphenoxy)benzol-sulfonylisocyanat | 2-Amino-4-methoxy-6-methylpyrimidin | N-[2-(2-Methoxyphenoxy)phenyl-sulfonyl]-N'-(4-methoxy-6-methyl-2-pyrimidyl)harnstoff |
| 24 | 2-(2,4-Dichlorophenoxy)-benzolsulfonylisocyanat | 2-Amino-4-methoxy-6-methyl-1,3,5-triazin | N-[2-(2,4-dichlorophenoxy)phenyl-sulfonyl]-N'-(4-methoxy-6-methyl-1,3,5-triazin-2-yl)harnstoff |

*Herstellung der Ausgangsstoffe*

Beispiel 25

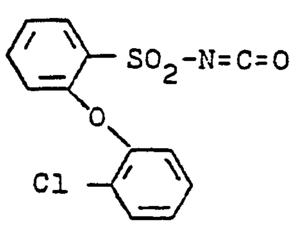

(II-1)

11,4 g 2-(2-Chlorophenoxy)benzolsulfonamid, 4 g n-Butylisocyanat und 0,5 g 1,4-Diazabicyclo[2,2,2]-octan wurde zu 50 ml Xylol gegeben, und die Mischung wurde 2 h unter Rühren zum Rückfluß erhitzt. Danach wurde die Innentemperatur auf 125°C gesenkt und eine Lösung von 6 g Trichloromethylchlorameisensäureester in 15 ml Xylol im Laufe von 2 h bei 120°C bis 125°C tropfenweise zu der vorgenannten Lösung hinzugefügt. Nach Beendigung der Zugabe wurde die Lösung 2 h erhitzt. Danach wurde die Lösung abgekühlt, unlösliche Bestandteile wurden abfiltriert, das Lösungsmittel wurde abdestilliert, und der Rückstand wurde destilliert, wonach 9,3 g des gewünschten Produkts, 2-(2-Chloro-phenoxy)benzolsulfonylisocyanat erhalten wurden; Sdp. 145°C bis 148°C bei 0,67 mbar (0,5 mmHg).

Weitere substituierte Benzolsulfonylisocyanate der Formel (II) gemäß der vorliegenden Erfindung, wie sie in der folgenden Tabelle 4 aufgeführt sind, wurden unter Verwendung der angegebenen Ausgangsstoffe entsprechend der in Beispiel, 25 beschriebenen Verfahrensweise hergestellt.

TABELLE 4

| Verbind. Nr. | Ausgangsstoff | Ausgangsstoff | Produkt |
|---|---|---|---|
| 26 | 2-(4-Chlorophenoxy)benzol-sulfonamid | Trichloromethylchlor-ameisensäureester | 2-(4-Chlorophenoxy)benzol-sulfonylisocyanat |
| 27 | 2-(2-Fluorophenoxy)benzol-sulfonamid | Phosgen | 2-(2-Fluorophenoxy)benzol-sulfonylisocyanat |
| 28 | 2-Chloro-6-phenoxybenzol-sulfonamid | Trichloromethylchlor-ameisensäureester | 2-Chloro-6-phenoxybenzol-sulfonylisocyanat |
| 29 | 2-Methoxy-5-phenylbenzol-sulfonamid | Phosgen | 2-Methoxy-5-phenylbenzol-sulfonylisocyanat |
| 30 | 2-Ethoxy-5-phenylbenzol-sulfonamid | Phosgen | 2-Ethoxy-5-phenylbenzol-sulfonylisocyanat |
| 31 | 2-(2-Methoxyphenoxy)-benzolsulfonamid | Phosgen | 2-(2-Methoxyphenoxy)benzol-sulfonylisocyanat |
| 32 | 2-(4-Nitrophenyl)benzol-sulfonamid | Trichloromethylchlor-ameisensäureester | 2-(4-Nitrophenyl)benzol-sulfonylisocyanat |
| 33 | 2-(o-Tolyloxy)benzol-sulfonamid | Phosgen | 2-(o-Tolyloxy)benzol-sulfonylisocyanat |
| 34 | 5-Methyl-2-phenoxybenzol-sulfonamid | Phosgen | 5-Methyl-2-phenoxybenzol-sulfonylisocyanat |
| 35 | 2-(4-Isopropylphenoxy)-benzolsulfonamid | Trichloromethylchlor-ameisensäureester | 2-(4-Isopropylphenoxy)benzol-sulfonylisocyanat |
| 36 | 2-Bromo-4-nitro-6-phenyl-benzolsulfonamid | Trichloromethylchlor-ameisensäureester | 2-Bromo-4-nitro-6-phenylbenzol-sulfonylisocyanat |
| 37 | 2-(2,4-Dichlorophenoxy)-benzolsulfonamid | Phosgen | 2-(2,4-Dichlorophenoxy)benzol-sulfonylisocyanat |

**Patentansprüche für die Vertragsstaaten: BE CH DE FR GB IT LI NL und SE**

1. Substituierte Phenylsulfonylharnstoffderivate der allgemeinen Formel I

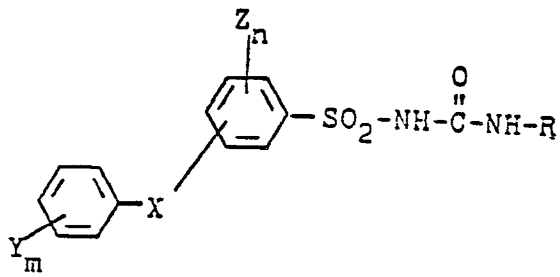

(I)

in der

X für ein Sauerstoff-Atom oder eine Einfachbindung steht,

Y und Z unabhängig voneinander ein Halogen-Atom, eine $C_1$- bis $C_6$-Alkyl-Gruppe, eine $C_1$- bis $C_6$-Alkoxy-Gruppe oder eine Nitro-Gruppe und

R eine Gruppe der allgemeinen Formel

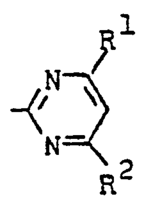

oder

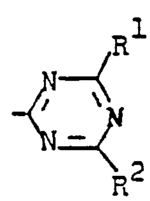

bezeichnen, in der

$R^1$ und $R^2$ unabhängig voneinander für eine $C_1$- bis $C_6$-Alkyl-Gruppe oder eine $C_1$ bis $C_6$-Alkoxy-Gruppe stehen, und

m und n jeweils unabhängig voneinander 0, 1 oder 2 sind, mit der Maßgabe, daß m und n nicht beide gleichzeitig 0 sind, daß Z — wenn n = 2 ist — unterschiedliche Substituenten in Form von Br und $NO_2$ bedeuten kann und daß — wenn X für eine Einfachbindung steht — entweder n = 2 ist oder der Rest

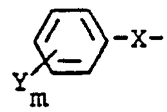

für eine 5-Phenyl- oder eine 2-(4-Nitrophenyl)-Gruppe steht.

2. Verbindungen nach Anspruch 1, dadurch gekennzeichnet, daß

X für ein Sauerstoff-Atom oder eine Einfachbindung,

Y und Z jeweils unabhängig voneinander für ein Fluor-, Chlor-, Brom- oder Iod-Atom oder eine Methyl-, Ethyl-, Propyl-, Isopropyl-, n- (iso-, sec- oder tert-)Butyl-, Methoxy-, Ethoxy-, Propoxy-, Isopropoxy-, n- (iso-, sec- oder tert-)Butoxy- oder Nitro- Gruppe und

R für eine 2-Pyrimidinyl-Gruppe oder eine 1,3,5-Triazin-2-yl-Gruppe, in denen die 4- und 6-Stellungen des Pyrimidin-Rings oder 1,3,5-Triazin-Rings durch Substituenten ausgewählt aus Methyl, Ethyl, Propyl, Isopropyl, n- (iso-, sec- oder tert-)Butyl, Methoxy, Ethoxy, Propoxy, Isopropoxy und n- (iso-, sec- oder tert-)Butoxy substituiert sind,

stehen und

m und n jeweils unabhängig voneinander 0, 1 oder 2 sind, mit der Maßgabe, daß m und n nicht beide gleichzeitig 0 sind, daß Z — wenn n = 2 ist — unterschiedliche Substituenten bedeuten kann und daß — wenn X für eine Einfachbindung steht — entweder n = 2 ist oder der Rest

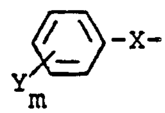

für eine 5-Phenyl- oder eine 2-(4-Nitrophenyl)-Gruppe steht.

3. Verbindung nach Anspruch 1 der Formel (1)

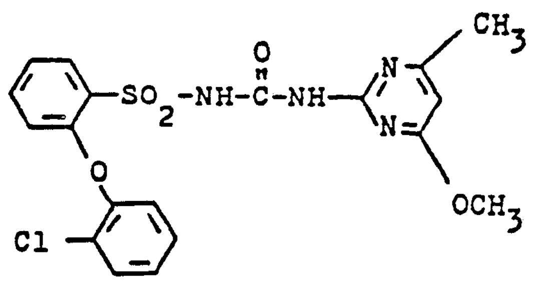

(1)

4. Verfahren zur Herstellung einer Verbindung nach Anspruch 1 bis 3, dadurch gekennzeichnet, daß eine Verbindung der allgemeinen Formel II

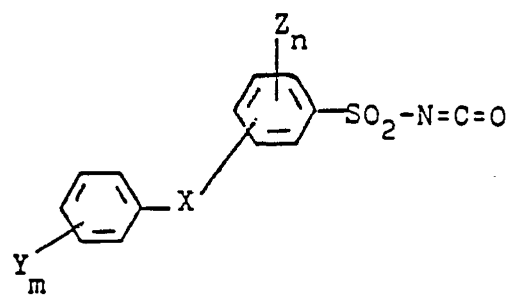

(II)

in der X, Y, Z, m und n die in Anspruch 1 angegebenen Bedeutungen haben, mit einer Verbindung der allgemeinen Formel III

$$H_2N—R \quad (III),$$

in der R die in Anspruch 1 angegebene Bedeutung hat, umgesetzt wird, gegebenenfalls in Gegenwart eines inerten organischen Lösungsmittels oder Verdünnungsmittels und/oder in Gegenwart eines Katalysators.

5. Substituiertes Benzolsulfonylisocyanat der allgemeinen Formel

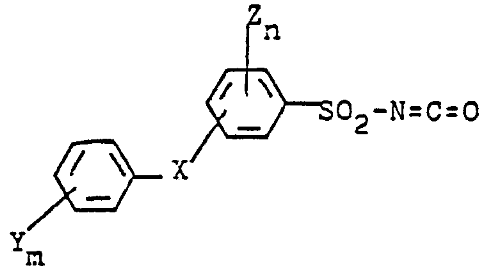

(II)

in der

X für ein Sauerstoff-Atom oder eine Einfachbindung steht,

Y und Z unabhängig voneinander ein Halogen-Atom, eine $C_1$- bis $C_6$-Alkyl-Gruppe, eine $C_1$- bis $C_6$-Alkoxy-Gruppe oder eine Nitro-Gruppe bezeichnen und

m und n jeweils unabhängig voneinander 0, 1 oder 2 sind, mit der Maßgabe, daß m und n nicht beide gleichzeitig 0 sind, daß Z — wenn n = 2 ist — unterschiedliche Substituenten in Form von Brund $NO_2$ bedeuten kann und daß — wenn X für eine Einfachbindung steht — entweder n = 2 ist oder der Rest

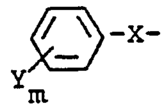

für eine 5-Phenyl- oder eine 2-(4-Nitrophenyl)-Gruppe steht.

6. Verfahren zur Herstellung einer Verbindung nach Anspruch 5, dadurch gekennzeichnet, daß substituierte Benzolsulfonamide der allgemeinen Formel IV

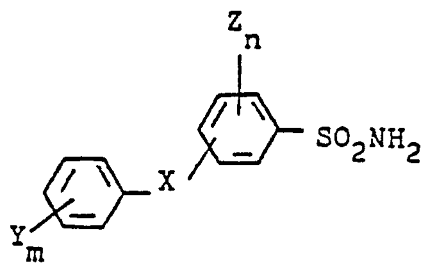

(IV)

in der

X, Y, Z, m und n die in Anspruch 5 angegebenen Bedeutungen haben, mit Phosgen ($COCl_2$) oder Trichloromethylchlorameisensäureester ($CCl_3OCOCl$), in Gegenwart eines Kohlenwasserstoff-Isocyanats, gegebenenfalls in Gegenwart eines inerten organischen Lösungsmittels oder Verdünnungsmittels und/oder in Gegenwart eines tertiären Amins als Katalysator, umgesetzt werden.

7. Herbizide Mittel, gekennzeichnet durch einen Gehalt an mindestens einem substituierten Phenylsulfonylharnstoff-Derivat der Formel (I) gemäß Anspruch 1.

8. Verwendung von substituierten Phenylsulfonylharnstoff-Derivaten der Formel (I) gemäß Anspruch 1 zur Bekampfung von unerwünschtem Pflanzenwachstum.

9. Verfahren zur Herstellung von herbiziden Mitteln, dadurch gekennzeichnet, daß man substituierte Phenylsulfonylharnstoff-Derivate der Formel (I) gemäß Anspruch 1 mit Streckmitteln und/oder oberflächenaktiven Mitteln vermischt.

**Claims for the Contracting States: BE CH DE FR GB IT LI NL and SE**

1. Substituted phenylsulphonylurea derivatives of the general formula I

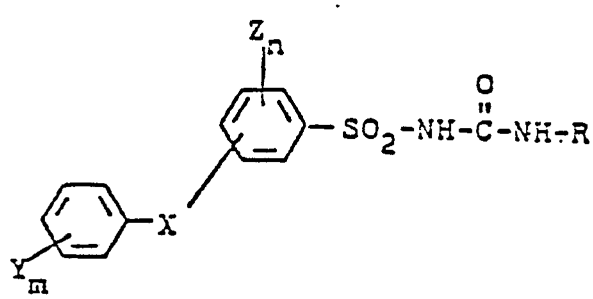

(I)

in which

X represents an oxygen atom or a single bond,

Y and Z independently of one another designate a halogen atom, a $C_1$ to $C_6$ alkyl group, a $C_1$ to $C_6$ alkoxy group or a nitro group and

R designates a group of the general formula

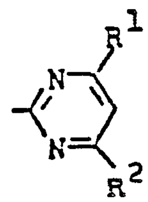

or

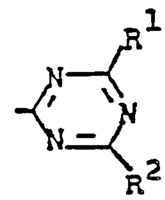

in which

$R^1$ and $R^2$ independently of one another represent a $C_1$ to $C_6$ alkyl group or a $C_1$ to $C_6$ alkoxy group, and

m and n each independently of one another are 0, 1 or 2, with the proviso that m and n are not both 0 at the same time, that Z can denote different substituents in the form of Br and $NO_2$ when n = 2 and that, when X represents a single bond, either n = 2 or the radical

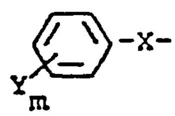

represents a 5-phenyl or a 2-(4-nitrophenyl) group.

2. Compounds according to Claim 1, characterised in that

X represents an oxygen atom or a single bond,

Y and Z each independently of one another represent a fluorine, chlorine, bromine or iodine atom or a methyl, ethyl, propyl, isopropyl, n- (iso-, sec- or tert-)butyl, methoxy, ethoxy, propoxy, isopropoxy, n- (iso-, sec- or tert-)butoxy or nitro group and

R represents a 2-pyrimidinyl group or a 1,3,5-triazin-2-yl group, in which the 4 and 6 positions of the pyrimidine ring or 1,3,5-triazine ring are substituted by substituents selected from methyl, ethyl, propyl, isopropyl, n- (iso-, sec- or tert-)butyl, methoxy, ethoxy, propoxy, isopropoxy and n- (iso, sec- or tert-)butoxy, and

m and n each independently of one another are 0, 1 or 2 with the proviso that m and n are not both 0 at the same time, that Z can denote different substituents when n = 2 and that, when X represents a single bond, either n = 2 or the radical

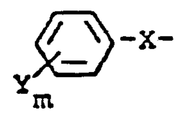

represents a 5-phenyl or a 2-(4-nitrophenyl) group.

3. Compound according to Claim 1 of the formula (I)

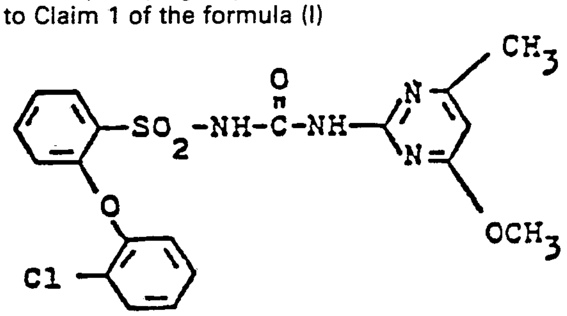

4. Process for the preparation of a compound according to Claim 1 to 3, characterised in that a compound of the general formula II

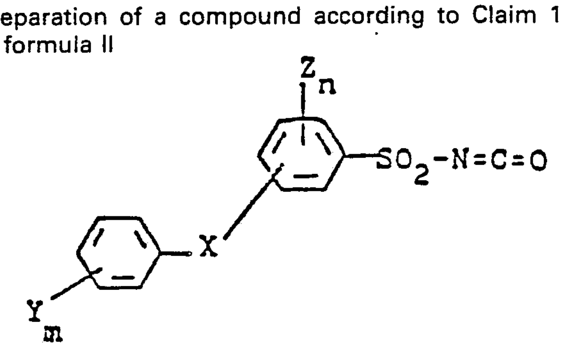

(II)

in which

X, Y, Z, m and n have the meanings given in Claim 1, is reacted with a compound of the general formula III

$$H_2N—R \quad (III),$$

in which

R has the meaning given in Claim 1, if appropriate in the presence of an inert organic solvent or diluent and/or in the presence of a catalyst.

5. A substituted benzenesulphonyl isocyanate of the general formula

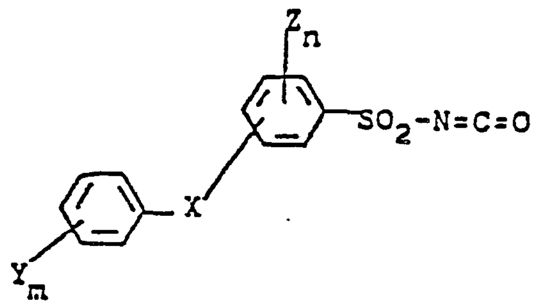

(II)

in which

X represents an oxygen atom or a single bond,

Y and Z independently of one another designate a halogen atom, a $C_1$ to $C_6$ alkyl group, a $C_1$ to $C_6$ alkoxy group or a nitro group and

m and n each independently of one another are 0, 1 or 2, with the proviso that m and n are not both 0 at the same time, that Z can denote different substituents in the form of Br and $NO_2$ when n = 2 and that, when X represents a single bond, either n = 2 or the radical

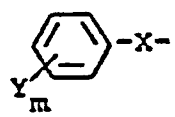

represents a 5-phenyl or a 2-(4-nitrophenyl) group.

6. Process for the preparation of a compound according to Claim 5, characterised in that substituted benzenesulphonamides of the general formula IV

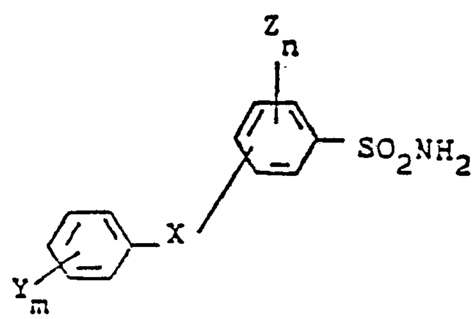

(IV)

in which

X, Y, Z, m and n have the meanings given in Claim 5, are reacted with phosgene ($COCl_2$) or trichloromethyl chloroformic acid ester ($CCl_3OCOCl$), in the presence of a hydrocarbon isocyanate, if appropriate in the presence of an inert organic solvent or diluent and/or in the presence of a tertiary amine as the catalyst.

7. Herbicidal agents, characterised in that they contain at least one substituted phenylsulphonylurea derivative of the formula (I) according to Claim 1.

8. Use of substituted phenylsulphonylurea derivatives of the formula (I) according to Claim 1 for combating undesired plant growth.

9. Process for the preparation of herbicidal agents, characterised in that substituted phenylsulphonylurea derivatives of the formula (I) according to Claim 1 are mixed with extenders and/or surface-active agents.

**Revendications pour les Etats contractants: BE CH DE FR GB IT LI NL et SE**

1. Dérivés substitués de phénylsulfonylurée de formule générale I

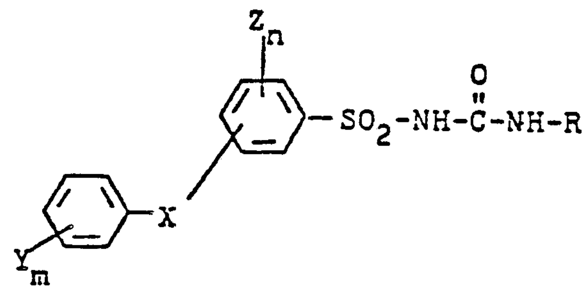

(I)

dans laquelle

X représente un atome d'oxygène ou une liaison simple,

Y et Z représentent, indépendamment l'un de l'autre, un atome d'halogène, un groupe alkyle en $C_1$ à $C_6$, un groupe alkoxy en $C_1$ à $C_6$ ou un groupe nitro et

R est un groupe de formule générale

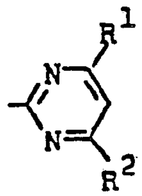

ou

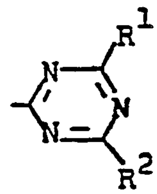

dans laquelle

$R^1$ et $R^2$ représentent, indépendamment l'un de l'autre un groupe alkyle en $C_1$ à $C_6$ ou un groupe alkoxy en $C_1$ à $C_6$, et

*m* et *n* ont chacun, indépendamment l'un de l'autre, la valeur 0, 1 ou 2, sous réserve que *m* et *n* ne soient pas tous deux en même temps égaux à 0, que Z, lorsque *n* est égal à 2, puisse désigner des substituants différents sous la forme de Br et $NO_2$ et que — lorsque X représente une liaison simple — *n* soit égal à 2 ou le reste

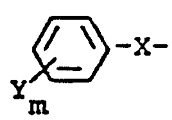

soit un groupe 5-phényle ou un groupe 2-(4-nitrophényle).

2. Composée suivant la revendication 1, caractérisés en ce que

X représente un atome d'oxygène ou une liaison simple,

Y et Z représentent chacun, indépendamment l'un de l'autre, un atome de fluor, de chlore, de brome ou d'iode ou un groupe méthyle, éthyle, propyle, isopropyle, n-butyle, isobutyle, sec.-butyle ou tertio-butyle, méthoxy, éthoxy, propoxy, isopropoxy, n-butoxy, isobutoxy, sec.-butoxy, tertio-butoxy ou nitro et

R désigne un groupe 2-pyrimidinyle ou un groupe 1,3,5-triazine-2-yle dans lesquels les positions 4 et 6 du noyau de pyrimidine ou du noyau de 1,3,5-triazine sont substituées par des substituants choisis entre les groupes méthyle, éthylé, propyle, isopropyle, n-butyle, isobutyle, sec.-butyle, tertio-butyle, méthoxy, éthoxy, propoxy, isopropoxy, n-butoxy, isobutoxy, sec.-butoxy ou tertio-butoxy, et

*m* et *n* ont chacun, indépendamment l'un de l'autre, la valeur 1, 2 ou 3, sous réserve que *m* et *n* ne soient pas égaux à O tous deux en même temps, que Z — lorsque *n* est égal à 2 — puisse représenter des substituants différents et que — lorsque X est une liaison simple — *n* soit égal à 2 ou bien le reste

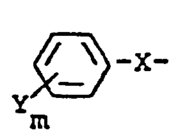

représente un groupe 5-phényle ou un groupe 2-(4-nitrophényle).

3. Composé suivant la revendication 1 de formule (1)

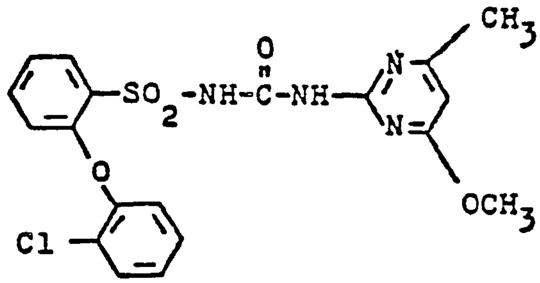

(1)

4. Procédé de production d'un composé suivant les revendications 1 à 3, caractérisé en ce qu'on fait réagir un composé de formule générale II

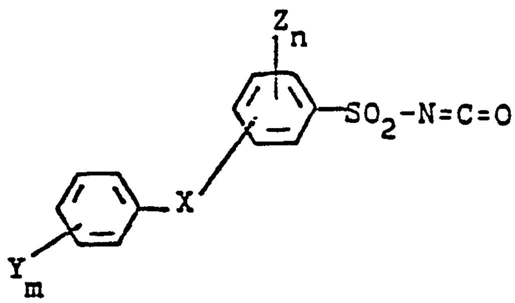

(II)

dans laquelle X, Y, Z, *m* et *n* ont les définitions indiquées dans la revendications 1, avec un composé de formule générale III

$$H_2N—R \quad (III),$$

dans laquelle R a la définition indiquée dans la revendication 1, éventuellement en présence d'un solvant ou diluant organique inerte et/ou en présence d'un catalyseur.

5. Benzènesulfonylisocyanate substitué de formule générale

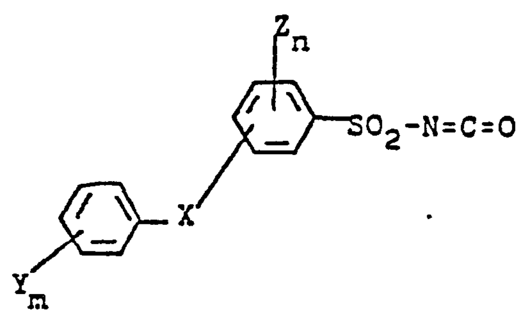

(II)

dans laquelle

X désigne un atome d'oxygène ou une liaison simple,

Y et Z représentent, indépendamment l'un de l'autre, un atome d'halogène, un groupe alkyle en $C_1$ à $C_6$, un groupe alkoxy en $C_1$ à $C_6$ ou un groupe nitro et

*m* et *n* ont chacun, indépendamment l'un de l'autre, la valeur 0, 1 ou 2, sous réserve que *m* et *n* ne soient pas tous deux en même temps égaux à 0, que Z — lorsque *n* est égal à 2 — Puisse désigner des substituants différents sous forme de Br et $NO_2$ et que — lorsque X est une liaison simple — *n* soit égal à 2 ou le reste

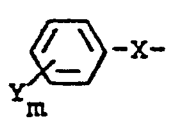

représente un reste 5-phényle ou un reste 2-(4-nitrophényle).

6. Procédé de production d'un composé suivant la revendication 5, caractérisé en ce qu'on fait réagir des benzènesulfonamides substitués de formule générale

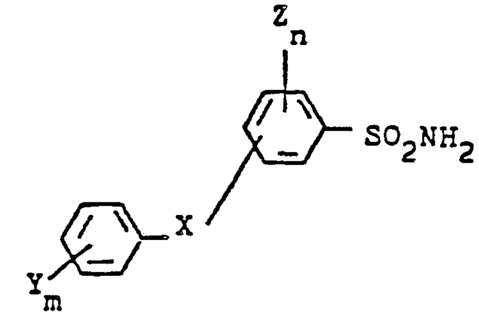

(IV)

dans laquelle

X, Y, Z, *m* et *n* ont les définitions indiquées dans la revendication 5, avec le phosgène ($COCl_2$) ou l'ester trichlorométhylique de l'acide chloroformique ($CCl_3OCOCl$), en présence d'un isocyanate d'hydrocarbure, éventuellement en présence d'un solvant ou d'un diluant organique inerte et/ou en présence d'une amine tertiaire comme catalyseur.

7. Compositions herbicides, caractérisées par une teneur en au moins un dérivé substitué de phénylsulfonylurée de formule (I) suivant la revendication 1.

8. Utilisation de dérivés substitués de phénylsulfonylurée de formule (I) suivant la revendication 1 pour combattre une végétation indésirable.

9. Procédé de préparation de compositions herbicides, caractérisé en ce qu'on mélange des dérivés substitués de phénylsulfonylurée de formule (I) suivant la revendication 1 avec des diluants et/ou des agents tensio-actifs.

**Beschreibung für den Vertragsstaat: AT**

Die vorliegende Erfinding betrifft ein Verfahren zur Herstellung bestimmter neuer substituierter Phenylsulfonylharnstoff-Derivate, und deren Verwendung als Herbizide sowie die Beschreibung bestimmter substituierter Benzolsulfonylisocyanate als Zwischenprodukte bei deren Herstellung.

In US—A—4 127 405 und US—A—4 169 719 und in der veröffentlichten japanischen Patentanmeldung Nr. 52—122384 ist offenbart, daß Verbindungen der allgemeinen Formel

$$R_1{-}SO_2{-}NH{-}\overset{\overset{\displaystyle W}{\|}}{C}{-}NH{-}R \quad (V),$$

R 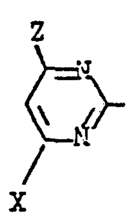 oder 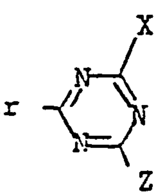 und R1 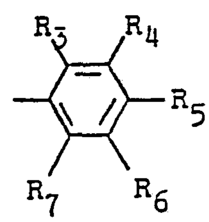 , 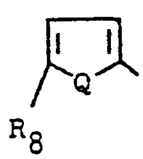 oder 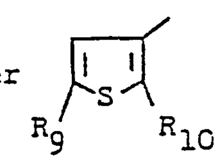

bezeichnen, worin

$R_3$ und $R_6$ unabhängig voneinander ein Wasserstoff-, Fluor-, Brom- oder Iod-Atom, eine Alkyl-Gruppe mit 1 bis 4 Kohlenstoff-Atomen, eine Alkoxy-Gruppe mit 1 bis 4 Kohlenstoff-Atomen, eine Nitro-, Trifluoromethyl- oder Cyano-Gruppe oder eine $CH_3S(O)_n$- oder $CH_3CH_2S(O)_n$-Gruppe (worin n 0, 1 oder 2 ist),

$R_4$ ein Wasserstoff-, Fluor-, Chlor-, Brom-Atom oder eine Methyl-Gruppe,

$R_5$ ein Wasserstoff-, Fluor-, Chlor-, Brom-Atom, eine Methyl-Gruppe oder eine Methoxy-Gruppe,

$R_7$ ein Wasserstoff-, Fluor- oder Brom-Atom, eine Alkyl-Gruppe mit 1 oder 2 Kohlenstoff-Atomen oder eine Alkoxy-Gruppe mit 1 oder 2 Kohlenstoff-Atomen,

$R_8$, $R_9$ und $R_{10}$ unabhängig voneinander ein Wasserstoff-, Chlor- oder Brom-Atom oder eine Methylgruppe,

W und Q unabhängig voneinander ein Sauerstoff- oder Schwefel-Atom,

X ein Wasserstoff-, Chlor- oder Brom-Atom, eine Methyl- oder Ethyl-Gruppe, eine Alkoxy-Gruppe mit 1 bis 3 Kohlenstoff-Atomen, eine Trifluoromethyl-Gruppe oder $CH_3S$- oder $CH_3OCH_2$- und

Z eine Methyl- oder Methoxy-Gruppe

bezeichnen, mit den zusätzlichen Bedingungen, daß

a) wenn $R_5$ kein Wasserstoff-Atom ist, mindestens einer der Substituenten $R_3$, $R_4$, $R_6$ und $R_7$ kein Wasserstoff-Atom ist und mindestens zwei der Substituenten $R_3$, $R_4$, $R_6$ und $R_7$ Wasserstoff-Atome sein müssen,

b) wenn $R_5$ ein Wasserstoff-Atom ist und sämtliche Substituenten $R_3$, $R_4$, $R_6$ und $R_7$ keine Wasserstoff-Atome sind, sämtliche Substituenten $R_3$, $R_4$, $R_6$ und $R_7$ entweder ein Chlor-Atom oder eine Methyl-Gruppe sein müssen, und

c) wenn sowohl $R_3$ als auch $R_7$ Wasserstoff-Atome sind, mindestens einer der Substituenten $R_3$, $R_4$, $R_5$ und $R_6$ ein Wasserstoff-Atom sein muß.

und deren physiologisch unbedenkliche Salze herbizide Wirksamkeit besitzen.

Die herbiziden Eigenschaften dieser Verbindungen sind im Hinblick auf ihren Wirkungsspiegel und ihre Selektivität jedoch nicht immer zufriedenstellend.

In der vorgängigen, jedoch nicht vorveröffentlichten Patentanmeldung EP—A— 56 969 werden herbizid wirksame Verbindungen beschrieben, die den erfindungsgemäßen Verbindungen der nachfolgend angegebenen Formel (I) ähnlich sind; jedoch sind die beiden Benzolringe unsubstituiert (d.h. die Indices m und n stehen gleichzeitig für Null).

Aus der vorgängigen Patentanmeldung EP—A— 21 641 ist es bekannt, daß man die als Zwischenprodukte benötigten Arylsulfonylisocyanate vom Typ der nachfolgend angegebenen Formel (II) in guten Ausbeuten herstellen kann, indem man die entsprechenden Arylsulfonamide in Gegenwart jeweils katalytischer Mengen eines Kohlenwasserstoff-isocyanats und einer tertiären Aminbase phosgeniert.

Gegenstand der vorliegenden Erfindung ist nunmehr, die Herstellung neuer Verbindungen, nämlich substituierter Phenylsulfonylharnstoff-Derivate der allgemeinen Formel

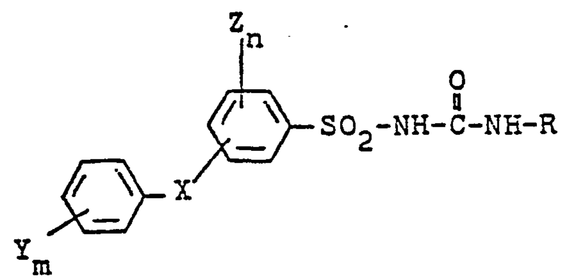

(I)

in der

X für ein Sauerstoff-Atom oder eine Einfachbindung steht,

Y und Z unabhängig voneinander ein Halogen-Atom, eine $C_1$- bis $C_6$-Alkyl-Gruppe, eine $C_1$- bis $C_6$-Alkoxy-Gruppe oder eine Nitro-Gruppe und

R eine Gruppe der allgemeinen Formel

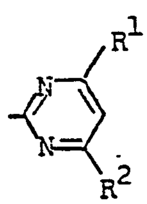

oder

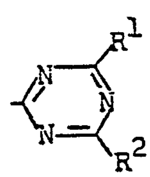

bezeichnen, in der

$R^1$ und $R^2$ unabhängig voneinander für eine $C_1$- bis $C_6$-Alkyl-Gruppe oder eine $C_1$- bis $C_6$-Alkoxy-Gruppe stehen, und

m und n jeweils unabhängig voneinander 0, 1 oder 2 sind, mit der Maßgabe, daß m und n nicht beide gleichzeitig O sind und daß Z — wenn n = 2 ist — unterschiedliche Substituenten in Form von Br und $NO_2$ bedeuten kann.

Gegenstand der vorliegenden Erfindung ist somit ein Verfahren zur Herstellung einer Verbindung der Formel (I) gemäß der vorliegenden Erfindung, das dadurch gekennzeichnet ist, daß eine Verbindung der allgemeinen Formel

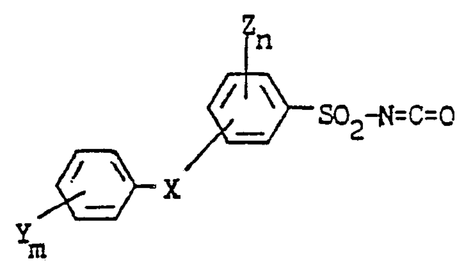

(II),

in der X, Y, Z, m und n die im Vorstehenden angegebenen Bedeutungen haben, mit einer Verbindung der allgemeinen Formel

$$H_2N—R \quad (III)$$

umgesetzt wird, in der R die vorstehende Bedeutung hat.

Gegenstand der vorliegenden Erfindung ist außerdem die Herstellung neuer Verbindungen, nämlich der substituierten Benzolsulfonylisocyanate der allgemeinen Formel

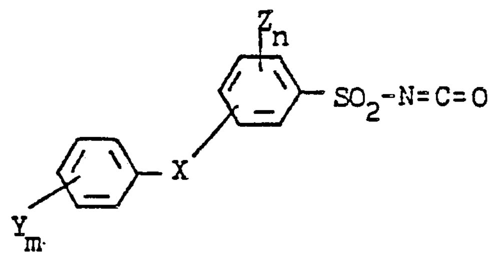

(II)

in der X, Y, Z, m und n die im Vorstehenden angegebenen Bedeutungen haben.

Gegenstand der vorliegenden Erfindung ist außerdem ein Verfahren zur Herstellung einer Verbindung der Formel (II) gemäß der vorliegenden Erfindung, das dadurch gekennzeichnet ist, daß ein substituiertes Benzolsulfonamid der allgemeinen Formel

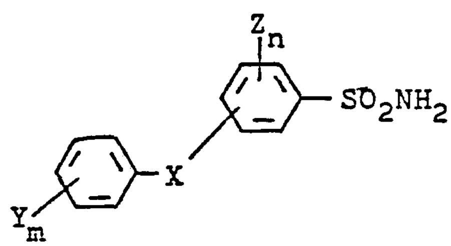

(IV)

in der X, Y, Z, m und n die im Vorstehenden angegebenen Bedeutungen haben, mit Phosgen ($COCl_2$) oder Trichloromethylchlorameisensäureester ($CCl_3OCOCl$) in Gegenwart eines Kohlenwasserstoff-isocyanats umgesetzt wird.

Nunmehr wurde gefunden, daß die substituierten Phenylsulfonylharnstoff-Derivate der Formel (I) eine hervorragende selektive herbizide Wirksamkeit besitzen, die aufgrund der Kenntnis der oben erwähnten Verbindungen gemäß dem Stand der Technik nicht zu erwarten war. Insbesondere zeigen die aktiven, gemäß der vorliegenden Erfindung hergestellten Verbindungen im Vergleich zu konventionellen Herbiziden, die eine beträchtliche Phytotoxizität gegenüber Reispflanzen aufweisen, auch wenn sie ihre herbizide Wirkung bereits bei bemerkenswert niedrigen Dosierungen entfalten, überhaupt keine Phytotoxizität gegen Reispflanzen und eine ausgezeichnete, selektive herbizide Wirkung bei niedrigen Dosierungen. Damit repräsentiert die vorliegende Erfindung einen erheblichen technischen Fortschritt.

Bevorzugte Verbindungen der Formel (I) sind diejenigen, in denen

X für ein Sauerstoff-Atom oder eine Einfachbindung,

Y und Z jeweils unabhängig voneinander für ein Fluor-, Chlor-, Brom- oder Iod-Atom oder eine Methyl-, Ethyl-, Propyl-, Isopropyl-, n- (iso-, sec- oder tert-)Butyl-, Methoxy-, Ethoxy-, Propoxy-, Isopropoxy-, n- (iso-, sec- oder tert-)Butoxy- oder Nitro-Gruppe und

R für eine 2-Pyrimidinyl-Gruppe oder eine 1,3,5-Triazin-2-yl-Gruppe, in denen die 4- und 6-Stellungen des Pyrimidin-rings oder 1,3,5-Triazin-Rings durch Substituenten ausgewählt aus Methyl, Ethyl, Propyl, Isopropyl, n- (iso-, sec- oder tert-)Butyl, Methoxy, Ethoxy, Propoxy, Isopropoxy und n- (iso-, sec- oder tert)-Butoxy substituiert sind,

stehen und

m und n jeweils unabhängig voneinander 0, 1 oder 2 sind, mit der Maßgabe, daß m und n nicht beide gleichzeitig O sind und daß Z — wenn n = 2 ist — unterschiedliche Substituenten in Form von Br und $NO_2$ bedeuten kann.

Das Verfahren gemäß der vorliegenden Erfindung zur Herstellung der Verbindungen der Formel (I) wird durch die folgende Gleichung beschrieben:

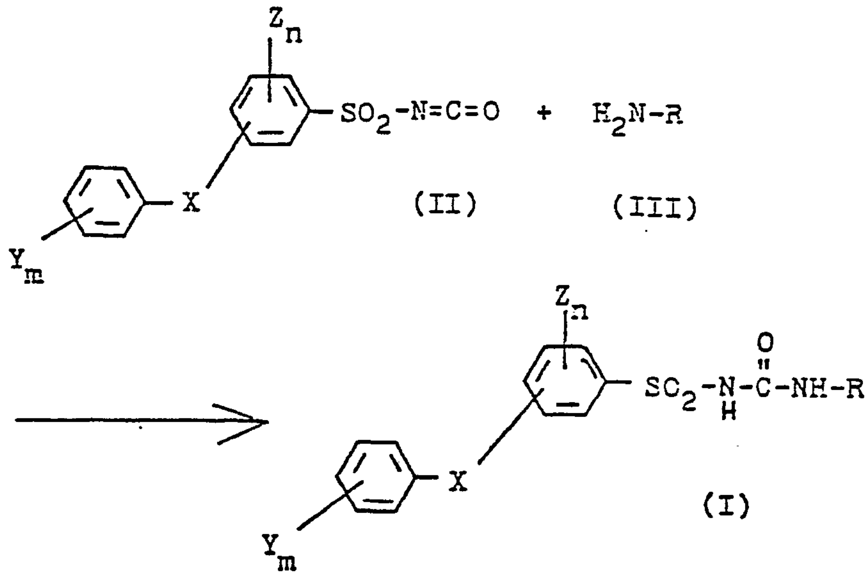

in der X, Y, Z, R, m und n die im vorstehenden angegebenen Bedeutungen haben.

Bevorzugte Ausgangsmaterialien der Formel (II) sind diejenigen, in denen X, Y, Z, m und n die in der Definition der bevorzugten Verbindungen der Formel (I) gemäß der vorliegenden Erfindung angegebenen bedeutungen haben.

Als Beispiele für die Verbindungen der Formel (II) seien erwähnt:
2-(2-Chlorophenoxy)benzolsulfonylisocyanat,
2-(4-Chlorophenoxy)benzolsulfonylisocyanat,
2-Methoxy-5-phenylbenzolsulfonylisocyanat,
2-(4-Nitrophenyl)benzolsulfonylisocyanat,
2-Bromo-4-nitro-6-phenylbenzolsulfonylisocyanat,
2-(2-Fluorophenoxy)benzolsulfonylisocyanat,
2-Chloro-6-phenoxybenzolsulfonylisocyanat,
2-(o-Tolyloxy)benzolsulfonylisocyanat,
5-Methyl-2-phenoxybenzolsulfonylisocyanat,
2-Ethoxy-5-phenylbenzolsulfonylisocyanat,
2-(4-Isopropylphenoxy)benzolsulfonylisocyanat,
2-Chloro-6-phenylbenzolsulfonylisocyanat,
2-(2-Methoxyphenoxy)benzolsulfonylisocyanat und
2-(2,4-Dichlorophenoxy)benzolsulfonylisocyanat.

Bevorzugte Ausgangsmaterialien der Formel (III) sind diejenigen, in denen R die in der Definition der bevorzugten Verbindungen der Formel (I) gemäß der vorliegenden Erfindung angegebene Bedeutung hat.

Als Beipspiele für die Verbindungen der Formel (III) seien erwähnt:
2-Amino-4-methoxy-6-methylpyrimidin,
2-Amino-4,6-dimethoxy-1,3,5-triazin,
2-Amino-4-methoxy-6-methyl-1,3,5-triazin,
2-Amino-4,6-dimethoxypyridin,
2-Amino-4,6-dimethylpyrimidin,
2-Amino-4,6-dimethyl-1,3,5-triazin,
2-Amino-4,6-diethoxy-1,3,5-triazin,
2-Amino-4,6-dipropylpyrimidin und
2-Amino-4-methyl-6-propoxy-1,3,5-triazin.

Wenn beispielsweise 2-(2-Chlorophenoxy)benzolsulfonylisocyanat und 2-Amino-4-methoxy-6-methylpyrimidin als Ausgangsmaterialien verwendet werden, wird das Verfahren gemäß der vorliegenden Erfindung zur Herstellung der Verbindungen der Formel (I) durch die folgende Gleichung beschrieben:

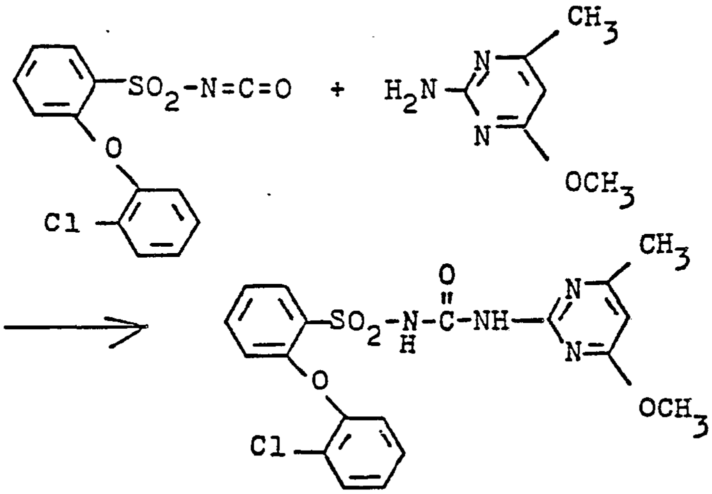

Das Verfahren zur Herstellung von Verbindungen der Formel (I) gemäß der vorliegenden Erfindung wird vorzugsweise unter Verwendung eines Lösungsmittels oder Verdünnungsmittels durchgeführt. Zu diesem Zweck können sämtliche inerten Lösungsmittel oder Verdünnungsmittel verwendet werden. Hierzu zählen insbesondere aliphatische, alicyclische und aromatische, gegebenenfalls chlorierte, Kohlenwasserstoffe (wie Hexan, Cyclohexan, Petrolether, Ligroin, Benzol, Toluol, Xylol, Methylchlorid, Chloroform, Kohlenstofftetrachlorid, Ethylenchlorid, Trichloroethylen und Chlorbenzol), Ether (wie Diethylether, Methylethylether, Di-isopropylether, Dibutylether, Propylenoxid, Dioxan und Tetrahydrofuran), Ketone (wie Aceton, Methylethylketon, Methylisopropylketon und Methylisobutylketon), Nitrile (wie Acetonitril, Propionitril und Acrylnitril), Ester (wie Ethylacetat und Amylacetat), Säureamide (wie Dimethylformamid und Dimethylacetamid), Sulfone und Sulfoxide (wie Dimethylsulfoxid), Sulfolan und Basen (wie Pyridin).

Dieses Verfahren wird zweckmäßigerweise in Gegenwart eines Katalysators wie 1,4-Diazabicyclo[2,2,2]octan durchgeführt.

Die Reaktionstemperatur kann innerhalb eines beträchtlichen Bereichs variiert werden. Im allgemeinen wird die Reaktion bei einer Temperatur im Bereich von −20°C und dem Siedepunkt der Reaktionsmischung, vorzugsweise zwischen 0°C und 100°C, durchgeführt.

Das Verfahren gemäß der vorliegenden Erfindung wird vorzugsweise unter dem Druck der Umgebung durchgeführt, wenngleich es auch bei erhöhtem oder vermindertem Druck durchgeführt werden kann.

Wie bereits im Vorstehenden erwähnt sind die substituierten Benzolsulfonylisocyanate der vorstehenden Formel (II) neue Verbindungen, die einen weiteren Gegenstand der vorliegenden Erfindung bilden und als Zwischenprodukte auf dem Wege zu den substituierten Phenylsulfonylharnstoff-Derivaten der vorstehenden Formel (I) wertvoll sind, die wie im Vorstehenden beschrieben eine hervorragende selektive herbizide Wirkung besitzen.

Das weitere Verfahren gemäß der vorliegenden Erfindung zur Herstellung der Ausgangsverbindungen der Formel (II) wird durch die folgende Gleichung beschrieben

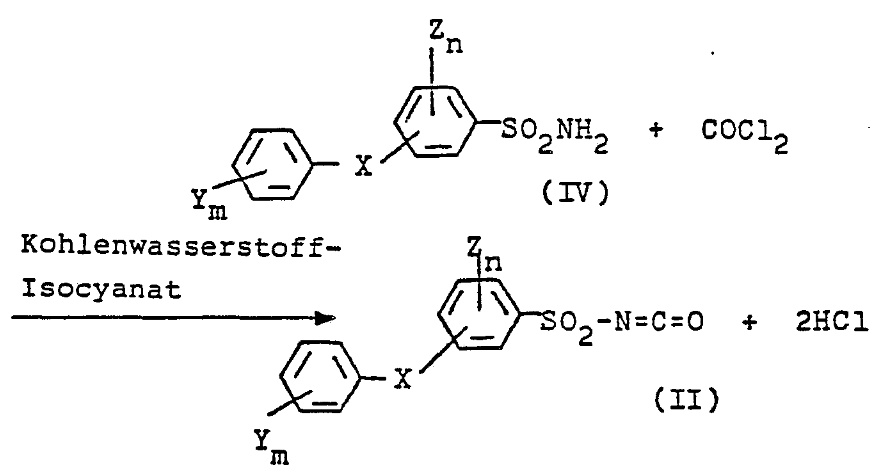


in der X, Y, Z, m und n die im Vorstehenden angegebenen Bedeutungen haben.

In dem vorstehenden Reaktionsschema kann anstelle des Phosgens ($COCl_2$) auch Trichloromethylchlorameisensäureester ($CCl_3OCOCl$) eingesetzt werden.

Spezielle Beispiele für das substituierte Benzolsulfonamid der Formel (IV), das ein Ausgangsstoff für die Herstellung des substituierten Benzolsulfonylisocyanats der Formel (II) ist, sind:

2-(2-Chlorophenoxy)benzolsulfonamid,
2-(4-Chlorophenoxy)benzolsulfonamid,
2-Methoxy-5-phenylbenzolsulfonamid,
2-(4-Nitrophenyl)benzolsulfonamid,
2-Bromo-4-nitro-6-phenylbenzolsulfonamid,
2-(2-Fluorophenoxy)benzolsulfonamid,
2-Chloro-6-phenoxybenzolsulfonamid,
2-(o-Tolyloxy)benzolsulfonamid,
5-Methyl-2-phenoxybenzolsulfonamid,
2-Ethoxy-5-phenylbenzolsulfonamid,
2-(4-Isopropylphenoxy)benzolsulfonamid,
2-Chloro-6-phenylbenzolsulfonamid,
2-(2-Methoxyphenoxy)benzolsulfonamid und
2-(2,4-Dichlorophenoxy)benzolsulfonamid

Die Reaktion zur Herstellung der Verbindungen der Formel (II) wird in Gegenwart eines Kohlenwasserstoff-Isocyanats durchgeführt, in dem der Kohlenwasserstoff-Rest eine Alkyl-Gruppe mit 3 bis 8 Kohlenstoff-Atomen oder eine Cycloalkyl-Gruppe mit 5 bis 8 Kohlenstoff-Atomen sein kann. Spezielle Beispiele für geeignete Kohlenwasserstoff-Isocyanate sind

n-Butylisocyanat,
n-Hexylisocyanat und
Cyclohexylisocyanat.

Wenn beispielsweise 2-(2-Chlorophenoxy)benzolsulfonamid, Trichloromethylchlorameisensäureester und n-Butylisocyanat als Reaktionsteilnehmer eingesetzt werden, wird das Verfahren gemäß der vorliegenden Erfindung zur Herstellung der Verbindungen der Formel (II) durch die folgende Gleichung beschrieben:

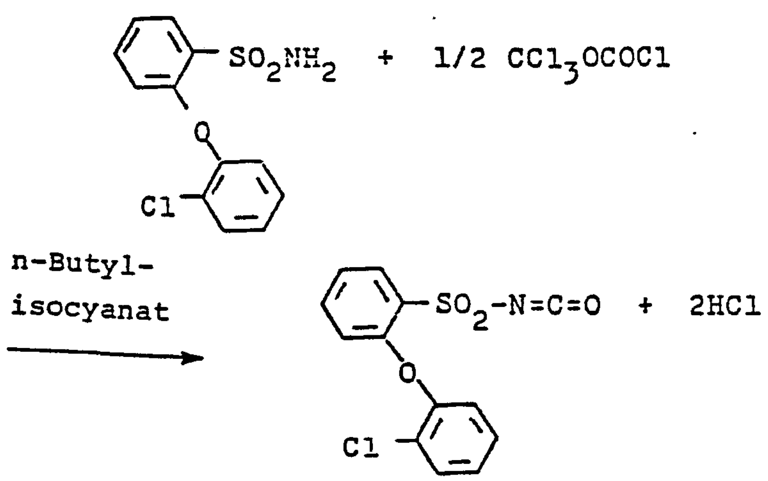


Das Kohlenwasserstoff-Isocyanat, das ein wesentlicher Reaktionsteilnehmer für die Erzeugung der Verbindungen der Formel (II) ist, kann am Ende der Reaktion zurückgewonnen werden, und infolgedessen ist das Verfahren für die Industrie vorteilhaft.

Das Verfahren zur Herstellung der Verbindungen der Formel (II) kann in jedem der inerten organischen Lösungsmittel oder Verdünnungsmittel durchgeführt werden, die weiter oben als Verdünnungsmittel bei der Herstellung der Verbindungen der Formel (I) genannt wurden.

Weiterhin wird das Verfahren zur Herstellung der Verbindungen der Formel (II) zweckmäßigerweise in Gegenwart eines Katalysators wie tertiärer Amine (beispielsweise Triethylamin, Dimethylcyclohexylamin und 1,4-Diazabicyclo[2,2,2]octan) durchgeführt.

Die Reaktionstemperatur kann innerhalb eines beträchtlichen Bereichs variiert werden. Im allgemeinen wird die Reaktion bei einer Temperatur im Bereich von −20°C und dem Siedepunkt der Reaktionsmischung, vorzugsweise zwischen 0°C und 100°C, durchgeführt.

Das Verfahren gemäß der vorliegenden Erfindung zur Herstellung der Verbindungen der Formel (II) wird vorzugsweise unter dem Druck der Umgebung durchgeführt, wenngleich es auch bei erhöhtem oder vermindertem Druck durchgeführt werden kann.

Die erfindungsgemäß hergstellten Wirkstoffe können als Mittel zur Bekämpfung von Unkräutern verwendet werden. Somit sind ein weiterer Gegenstand der Erfindung herbizide Mittel mit einem Gehalt am Verbindungen des Formel I sowie ein Verfahren Fur Herstellung dieser Mittel.

Unter "Unkräutern" im weitesten Sinne sind solche Pflanzen zu verstehen, die an Stellen wachsen, wo sie nicht erwünscht sind.

Die aktiven Verbindungen gemäß des Verfahrens der vorliegenden Erfindung sind hervorragend hinsichtlich ihrer Sicherheit, zeigen eine überlegene herbizide Wirkung und besitzen ein breites herbizides Spektrum.

Die vorstehend genanntten Verbindungen können beispielsweise zur Bekämpfung der folgenden Pflanzen verwendet werden:

Dikotyledonen-Unkräuter der Gattungen

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Rorippa, Rotala, Lindernia, Lamium, Veronica, Abutilon, Emex, Datura, Viola, Galeopsis, Papaver, Centaurea und Solanum; und

Monokotyledonen-Unkräuter der Gattungen

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Spenoclea, Dactyloctenium, Agrostis, Alopecurus und Apera.

Die aktiven Verbindungen gemäß des Verfahrens der vorliegenden Erfindung können beispielsweise als selektive Herbizide in den folgenden Kulturen verwendet werden:

Dikotyledonen-Kulturen der Gattungen

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita; und

Monokotyledonen-Kultaren der Gattungen

Oryza, Zea, Triticum, Hordeum, Avena, Secale, Sorghum, Panicum, Saccharum, Ananas, Asparagus und Allium.

Die Anwendung der erfindungsgemäß hergestellten, aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in einjährigen Kulturen verwendet werden.

Die aktiven Verbindungen gemäß des Verfahrens der vorliegenden Erfindung zeigen eine überlegene selektive Bekämpfungswirkung, insbesondere wenn sie als Mittel zur Bodenbehandlung vor der Keimung oder als Mittel zur Behandlung der Stengel und Blätter von Unkräutern und des Bodens auf Reisfeldern eingesetzt werden. Beispielsweise entfalten sie herbizide Wirkung gegen solche Unkräuter auf Reisfeldern wie die nachstehend aufgeführten, ohne daß sie irgend eine schädliche Wirkung auf die Reispflanzen ausüben:

Die Dikotyledonen-Unkräuter

Rotala indica Koehne,
Lindernia procumbens Philcox,
Ludwiga prostrata Roxburgh,
Potamogeton distinctus A. Bennett,
Elatine triandra Schkuhr

sowie

die Monokotyledonen-Unkräuter

Echinochloa crus-galli Beauvois var.,
Monochoria vaginalis Presl,
Eleocharis acicularis L.,
Eleocharis kuroguwai Ohwi,
Cyperus difformis L.,
Cyperus serotinus Rottboell,
Sagittaria pygmaea Miquel,
Alisma canaliculatum A. Braun et Bouché und
Scirpus juncoides Roxhurgh var.

Die Wirkstoffe können in übliche Präparate wie Lösungen, Emulsionen, Suspensionen, Pulver, Staübemittel, Schäume, Pasten, lösliche Pulver, Granulat, Aerosole, Suspensions-Emulsions-Konzentrate,

mit dem Wirkstoff imprägnierte natürliche und synthetische Materialien, sehr feine Kapseln in polymeren Substanzen, sowie ULV-Kaltvernebelungs- und Warmvernebelungspräparate umgewandelt werden.

Diese Präparate können in bekannter Weise hergestellt werden, beispielsweise durch Vermischen der Wirkstoffe mit Streckmitteln, d.h. flüssigen oder festen Verdünnungsmitteln oder Trägern, gegebenenfalls unter Verwendung von oberflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger, insbesondere als Lösungsmittel, eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen oder organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate und Arylsulfonate sowie Hydrolyse produkte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulut oder Latices, z.B. Gummiarabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe und Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,001 bis 100 Gewichts-%, vorzugsweise von 0,05 bis 95 Gewichts-% des Wirkstoffes.

Die Wirkstoffe gemäß des Verfahrens der vorliegenden Erfindung können in Präparaten oder verschiedenen Anwendungsformen als solche oder in Verbindung mit anderen aktiven Verbindungen wie Fungiziden, Bakteriziden, Insektiziden, Nematiziden, vogelabweisenden Mitteln, Wachstumsfaktoren, Pflanzennährstoffen sowie Mitteln zur Verbesserung der Bodenstruktur eingesetzt werden.

Insbesondere kann durch Zusatz geeigneter Mengen anderer Wirkstoffe zu den aktiven Herbiziden gemäß der vorliegenden Erfindung ein breiteres herbizides Spektrum und eine genauere Bekämpfungswirkung erreicht werden, und aufgrund einer solchen Vermischung kann auch eine synergistische Wirkung erreicht werden. Beispiele für solche anderen Wirkstoffe sind:

2-Chloro-2',6'-diethyl-N-(butoxymethyl)-acetanilid,
N-(O,O-Dipropyl-diethyl-phosphorylacetyl)-2-methylpiperidin,
S-(4-Chlorobenzyl)-N,N-diethylthiolcarbamat,
S-Ethyl-N,N-hexamethylenthiolcarbamat,
O-Methyl-O-(2-nitro-p-tolyl)-N-isopropylphosphoroamidthioat,
O-Ethyl-O-(2-nitro-5-methylphenyl)-N-sec-butylphosphoroamidthioat,
3,4-Dimethyl-2,6-dinitro-N-1-ethylpropylanilid,
α,α,α-Trifluoro-2,6-dinitro-N,N-dipropyl-p-toluidin,
2-Chloro-2',6'-diethyl-N-(n-propoxyethyl)-acetanilid,
4,5-Dichloro-1,3-thiazol-2-yloxyessigsäure-N-isopropyl-N-ethoxyethoxyamid,
5-Ethyl-1,3,4-thiadiazol-2-yloxyessigsäure-1',2',3',4'-tetrahydrochinolid,
Benzothiazol-2-yloxyessigsäure-N,N-diallylamid,
Benzoxazol-2-yloxyessigsäure-N-sec-butyl-N-methylamid,
Benzoxazol-2-yloxyessigsäure-N-cyclohexyl-N-methylamid,
Benzothiazol-2-yloxyessigsäure-N-methyl-N-(1-methylpropargyl)amid,
Benzoxazol-2-yloxyessigsäure-N-benzyl-N-propargylamid,
Benzothiazol-2-yloxyessigsäure-2'-ethylpiperidid,
Benzothiazol-2-yloxyessigsäure-2',4'-dimethylpiperidid,
Benzoxazol-2-yloxyessigsäure-2',4',6'-trimethylpiperid,
Benzoxazol-2-yloxyessigsäure-hexamethylenimid,
Benzothiazol-2-yloxyessigsäure-perhydroindolid,
Benzoxazol-2-yloxyessigsäure-perhydroindolid,

Benzothiazol-2-yloxyessigsäure-1′,2′,3′,4′-tetrahydrochinolid,
Benzoxazol-2-yloxyessigsäure-2′-methyl-1′,2′,3′4′-tetrahydrochinolid,
Benzoxazol-2-yloxyessigsäure-N-methylanilid,
Benzothiazol-2-yloxyessigsäure-N-methylanilid,
Benzoxazol-2-yloxyessigsäure-N-ethylanilid,
Benzoxazol-2-yloxyessigsäure-N-propylanilid,
Benzoxazol-2-yloxyessigsäure-isopropylanilid,
Benzothiazol-2-yloxyessigsäure-N-methyl-N-2′-methoxyanilid,
Benzoxazol-2-yloxyessigsäure-N-methyl-N-2′-methoxyanilid,
Benzoxazol-2-yloxyessigsäure-N-methyl-N-2′-trifluoromethylanilid,
Benzothiazol-2-yloxyessigsäure-N-methyl-N-2′-chloroanilid,
Benzoxazol-2-yloxyessigsäure-N-methyl-N-2′-chloroanilid,
Benzothiazol-2-yloxyessigsäure-N-methyl-N-2′-fluoroanilid,
Benzoxazol-2-yloxyessigsäure-N-methyl-N-2′-fluoroanilid,
Benzothiazol-2-yloxyessigsäure-N-methyl-N-3′-methylanilid,
Benzoxazol-2-yloxyessigsäure-N-methyl-N-3′-methylanilid,
Benzothiazol-2-yloxyessigsäure-N-methyl-N-3′-methoxyanilid,
Benzoxazol-2-yloxyessigsäure-N-methyl-N-3′-methyoxyanilid,
Benzothiazol-2-yloxyessigsäure-N-methyl-N-3′-isopropylanilid,
Benzoxazol-2-yloxyessigsäure-N-methyl-N-3′-isopropoxyanilid,
Benzothiazol-2-yloxyessigsäure-N-methyl-N-3′-trifluoromethylanilid,
Benzoxazol-2-yloxyessigsäure-N-methyl-N-3′-trifluoromethylanilid,
Benzothiazol-2-yloxyessigsäure-N-methyl-N-3′-chloroanilid,
Benzoxazol-2-yloxyessigsäure-N-methyl-N-3′-chloroanilid,
Benzothiazol-2-yloxyessigsäure-N-methyl-N-3′-fluoroanilid,
Benzoxazol-2-yloxyessigsäure-N-methyl-N-3′-fluoroanilid,
Benzothiazol-2-yloxyessigsäure-N-methyl-N-3′-bromoanilid,
Benzoxazol-2-yloxyessigsäure-N-methyl-N-3′-bromoanilid,
Benzoxazol-2-yloxyessigsäure-N-methyl-N-4′-methylanilid,
Benzoxazol-2-yloxyessigsäure-N-methyl-N-4′-methoxyanilid,
Benzoxazol-2-yloxyessigsäure-N-methyl-N-4′-fluoroanilid,
Benzoxazol-2-yloxyessigsäure-N-methyl-N-2′,3′-dimethylanilid,
Benzoxazol-2-yloxyessigsäure-N-methyl-N-2′,3′-dichloroanilid,
Benzoxazol-2-yloxyessigsäure-N-methyl-N-4′-chloro-2′-methylanilid,
Benzothiazol-2-yloxyessigsäure-N-methyl-N-2′,5′-dichloroanilid,
Benzoxazol-2-yloxyessigsäure-N-methyl-N-2′,5′-dichloroanilid,
Benzothiazol-2-yloxyessigsäure-N-methyl-N-3′,5′-dimethylanilid,
Benzoxazol-2-yloxyessigsäure-N-methyl-N-3′,5′-dimethylanilid
Benzoxazol-2-yloxyessigsäure-N-methyl-N-3′,5′-ditrifluoromethyl-anilid,
Benzoxazol-2-yloxyessigsäure-N-methyl-N-5′-indanylamid,
Benzothiazol-2-yloxyessigsäure-N-methyl-N-3′-ethylanilid,
Benzoxazol-2-yloxyessigsäure-N-methyl-N-3′-ethylanilid und
Benzothiazol-2-yloxyessigsäure-N-isopropylanilid.

Die Wirkstoffe können als solche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Besässern, Sprühen, Zerstäuben, Streuen oder Stäuben.

Die herbiziden Mittel gemäß der vorliegenden Erfindung können vor oder nach dem Auflaufen der Pflanzen zur Anwendung gelangen, werden jedoch vorzugsweise vor dem Auflaufen der Pflanzen, d.h. mittels des Verfahrens der Vorauflaufbehandlung, angewandt. Sie können auch vor der Einsaat in den Boden eingearbeitet werden.

Es ist auch möglich, die aktiven Verbindungen mittels des ULV-Verfahrens (ultra-low-volume method) zur Anwendung zu bringen, wodurch es möglich wird, die Verbindungen in einer Konzentration bis zu 100 Gew.-% einzusetzen.

Im praktischen Einsatz beträgt der Gehalt der Wirkstoffe in den verschiedenen Anwendungsformen oder gebrauchsfertigen Präparaten im allgemeinen von 0,01 bis 95 Gewichts-%, vorzugsweise von 0,05 bis 60 Gewichts-%.

Die flächenbezogenen Aufwandsmengen der aktiven Verbindung betragen im allgemeinen 0,1 bis 3 kg/ha, vorzugsweise 0,2 bis 1 kg/ha. In besonderen Fällen ist es jedoch möglich oder manchmal sogar notwendig, auch eine höhere oder niedrigere Menge außerhalb des genannten Bereichs aufzuwenden.

Die vorliegende Erfindung umfaßt somit herbizide Mittel, die als Wirkstoff eine Verbindung gemäß der vorliegenden Erfindung im Gemisch mit einem festen oder aus einem verflüssigter Gas bestehenden Verdünnungsmittel oder Träger oder im Gemisch mit einem ein oberflächenaktives Mittel enthaltenden flüssigen Verdünnungsmittel oder Träger enthält.

Die vorliegende Erfindung umfaßt weiter ein Verfahren zur Unkrautbekämpfung, das dadurch gekennzeichnet ist, daß eine Verbindung gemäß der vorliegenden Erfindung allein oder in Form eines Präparats, das als Wirkstoff eine Verbindung gemäß der vorliegenden Erfindung im Gemisch mit einem Verdünnungsmittel oder Träger enthält, auf die Unkräuter oder deren Lebensraum aufgebracht wird.

Die vorliegende Erfindung ist ferner auf Nutzpflanzen gerichtet, die dadurch gegen Schäden durch Unkräuter geschützt sind, daß sie auf Flächen angebaut werden, auf die unmittelbar vor und/oder während der Zeit des Anbauens eine Verbindung gemäß der vorliegenden Erfindung allein oder im Gemisch mit einem Verdünnungsmittel oder Träger aufgebracht wurde.

Es wird ersichtlich werden, daß die üblichen Methoden der Erzeugung geernteter Nutzpflanzen durch die vorliegende Erfindung verbessert werden können.

Zusammensetzungen gemäß der vorliegenden Erfindung werden in den folgenden Beispielen (i) bis (vi) erläutert. Hierin werden die Verbindungen der vorliegenden Erfindung durch die (in Klammer angegebene) Zahl des betreffenden Herstellungsbeispiels bezeichnet.

Beispiel i

15 Gew.-Teile der Verbindung (1), 80 Gew.-Teile eines 1:5-Gemisches aus pulvriger Diatomeenerde und Tonpulver, 2 Gew.-Teile Natriumalkylbenzolsulfonat und 3 Gew.-Teile Natriumalkylnaphthalinsulfonat/Formaldehyd-Kondensat wurden vermahlen und zu einem benetzbaren Pulver vermischt. Vor dem Spritzen wurde es mit Wasser verdünnt.

Beispiel ii

10 Gew.-Teile der Verbindung (2), 75 Gew.-Teile Dimethylformamid, 8 Gew.-Teile Polyoxyethylenalkylphenylether und 7 Gew.-Teile Calciumalkylbenzolsulfonat wurden unter Rühren miteinander vermischt, wodurch eine Emulsion erhalten wurde. Vor dem Spritzen wurde sie mit Wasser verdünnt.

Beispiel iii

2 Gew.-Teile der Verbindung (3) und 98 Gew.-Teile Tonpulver wurden vermahlen und gemischt, wodurch ein Stäubemittel erhalten wurde.

Beispiel iv

1,5-Gew.-Teile der Verbindung (4), 0,5 Gew.-Teile Isopropylhydrogenphosphat (PAP) und 98 Gew.-Teile Tonpulver wurden vermahlen und gemischt, wodurch ein Stäubemittel erhalten wurde.

Beispiel v

10 Gew.-Teile der Verbindung (5), 30 Gew.-Teile Bentonit (Montmorillonit), 58 Gew.-Teile Talkum und 2 Gew.-Teile Ligninsulfonat-Salz wurden miteinander vermischt, und nach Zusatz von 25 Gew.-Teilen Wasser wurde das Gemisch gut durchgemischt. Die erhaltene Mischung wurde mittels eines Extruder-Granulators zu einen Granulat mit einer Korngröße von 0,43 bis 2,0 mm (10 bis 40 mesh) verarbeitet und bei 40°C bis 50°C getrocknet, wodurch ein Granulat erhalten wurde. Dieses wurde durch Streuen aufgebracht.

Beispiel vi

95 Gew.-Teile Tonmineral-Teilchen mit einer Teilchengrößen-Verteilung im Bereich von 0,2 bis 2 mm wurden in einen Drehmischer gegeben, mit 5 Gew.-Teilen der Verbindung (6) durch Aufspritzen einer Lösung der Verbindung (6) in einem organischen Lösungsmittel gleichmäßig benetzt und dann bei 40°C bis 50°C getrocknet, wodurch ein Granulat erhalten wurde. Dieses wurde durch Streuen aufgebracht.

Die herbizide Wirkung der Verbindungen gemäß der vorliegenden Erfindung wird durch das folgende Biotest-Beispiel aufgezeigt.

In diesem Beispiel werden die Verbindungen gemäß der vorliegenden Erfindung jeweils durch die (in Klammer angegebene) Zahl des betreffenden Herstellungsbeispiels bezeichnet.

Die bekannte Vergleichsverbindung wird durch die nachstehenden Formel beschrieben:

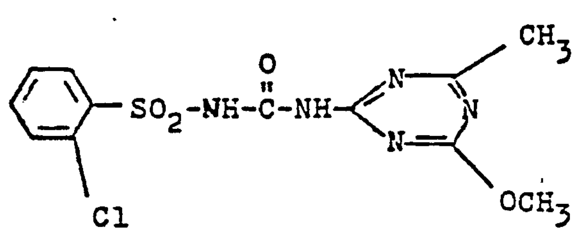

(V-1)

N-2-Chlorophenylsulfonyl-N'-(4-methoxy-6-methyl-1,3,5-triazin-2-yl)harnstoff
(eine in der veröffentlichten japanischen Patentanmeldung Nr. 52—122384 beschriebene Verbindung).

Beispiel A

Prüfung der Wirkung gegen im Wasser wachsende Reisfeld-Unkräuter durch Behandlung des Bodens sowie der Stengel und Blätter unter Bewässerungs-Bedingungen (Topf-Test):

Lösungsmittel: 5 Gewichtsteile Aceton;
Emulgator: 1 Gewichtsteil Benzyloxypolyglycolether.

Zur Herstellung eines geeigneten Wirkstoff-Präparats wurde 1 Gew.-Teil der aktiven Verbindung mit der vorbezeichneten Menge Lösungsmittel vermischt; die angegebene Menge Emulgator wurde zugesetzt, und das Konzentrat wurde mit Wasser auf die gewünschte Konzentration verdünnt.

Reis-Kulturboden wurde in Wagner-Töpfe (2 $dm^2$) gefüllt, und zwei Reis-Setzlinge (Varietät: Kinmaze) im 2- bis 3-Blattstadium (Pflanzenhöhe etwa 10 cm) wurden in jeden Topf umgepflanzt. Samen von Echinochloa crus-galli Beauvois var., Cyperus iria L., Monochoria vaginalis Presl, Scirpus juncoides Roxburgh var. und breitblättrigen Unkräutern, Kleine Stücke von Eleocharis acicularis L. und Knollen von Cyperus serotinus Rottboell und Sagittaria pygmaea Miquel wurden eingesetzt, und die Töpfe wurden in feuchtem Zustand gehalten. Als Echinochloa crus-galli Beauvois var. etwa bis zum 2-Blatt-Stadium gewachsen war (etwa 7 bis 9 Tage nach der Einsaat), wurden die Töpfe mit Wasser bis zu einer Tiefe von etwa 6 cm gefüllt, und eine vorher festgelegte Menge des Wirkstoffs wurde in Form einer Emulsion mittels einer Pipette dem Wasser zugegeben. Nach der Behandlung wurde das Wasser im Laufe von zwei Tagen mit einer Geschwindigkeit von 2 bis 3 cm pro Tag aus den Töpfen heraussickern gelassen. Danach wurde die Wassertiefe in den Töpfen bei etwa 3 cm gehalten. Vier Wochen nach der chemischen Behandlung wurden die herbizide Wirkung und der Grad der Phytotoxizität mit Hilfe einer Skala von 0 bis 10 nach dem folgenden Maßstab bewertet.

Die Wirkungen wurden als Unkrautvernichtungsrate mit Hilfe der nachstehenden Skala von 0 bis 10 im Vergleich zu unbehandelten Kontrollpflanzen bewertet:

| Bewertung | Unkrautvernichtungsrate (bezogen auf die unbehandelten Kontrollpflanzen) |
|---|---|
| 10 | 100% (verdorrt) |
| 9 | mindestens 90%, jedoch weniger als 100% |
| 8 | mindestens 80%, jedoch weniger als 90% |
| 7 | mindestens 70%, jedoch weniger als 80% |
| 6 | mindestens 60%, jedoch weniger als 70% |
| 5 | mindestens 50%, jedoch weniger als 60% |
| 4 | mindestens 40%, jedoch weniger als 50% |
| 3 | mindestens 30%, jedoch weniger als 40% |
| 2 | mindestens 20%, jedoch weniger als 30% |
| 1 | mindestens 10%, jedoch weniger als 20% |
| 0 | weniger als 10% (unwirksam) |

Die Phytotoxität wurde mit Hilfe der nachstehenden Skala von 0 bis 10 im Vergleich zu unbehandelten Kontrollpflanzen bewertet:

| Bewertung | Phytotoxizitätsrate (bezogen auf die unbehandelten Kontrollpflanzen) |
|---|---|
| 10 | mindestens 90% (Ausrottung) |
| 9 | mindestens 80%, jedoch weniger als 90% |
| 8 | mindestens 70%, jedoch weniger als 80% |
| 7 | mindestens 60%, jedoch weniger als 70% |
| 6 | mindestens 50%, jedoch weniger als 60% |
| 5 | mindestens 40%, jedoch weniger als 50% |
| 4 | mindestens 30%, jedoch weniger als 40% |
| 3 | mindestens 20%, jedoch weniger als 30% |
| 2 | mindestens 10%, jedoch weniger als 20% |
| 1 | mehr als 0%, jedoch weniger als 10% |
| 0 | 0% (keine Phytotoxizität) |

Die Testergebnisse sind in Tabelle 1 aufgeführt.

TABELLE 1

| Verbindung | Wirkstoff-Menge kg/ha | Herbizide Wirkung Unkräuter A | B | C | D | E | F | G | H | Phytotoxität gegen Reis |
|---|---|---|---|---|---|---|---|---|---|---|
| (1) | 0,2 | 10 | 10 | 10 | 10 | 10 | 10 | 10 | 10 | 0 |
| (2) | 0,2 | 10 | 10 | 10 | 10 | 10 | 10 | 10 | 10 | 0 |
| (3) | 0,2 | 10 | 10 | 10 | 10 | 10 | 10 | 10 | 10 | 0 |
| (5) | 0,2 | 10 | 10 | 10 | 10 | 10 | 10 | 10 | 10 | 0 |
| (16) | 0,2 | 10 | 10 | 10 | 10 | 10 | 10 | 10 | 10 | 0 |
| (17) | 0,2 | 10 | 10 | 10 | 10 | 10 | 10 | 10 | 10 | 0 |
| (18) | 0,2 | 10 | 10 | 10 | 10 | 10 | 10 | 10 | 10 | 0 |
| (20) | 0,2 | 10 | 10 | 10 | 10 | 10 | 10 | 10 | 10 | 0 |
| (22) | 0,2 | 10 | 10 | 10 | 10 | 10 | 10 | 10 | 10 | 0 |
| (23) | 0,2 | 10 | 10 | 10 | 10 | 10 | 10 | 10 | 10 | 0 |
| (V—1) | 0,2 | 10 | 9 | 10 | 10 | 10 | 10 | 5 | 10 | 9 |

*Anmerkung:*

Die Symbole A bis H bezeichnen die nachstehenden Unkräuter:

A: Echinochloa crus-galli Beauv.var.
B: Eleocharis acicularis L.
C: Cyperus iria L.
D: Scirpus juncoides Roxburgh var.
E: Monochoria vaginalis Presl
F: Breitblättrige Unkräuter (darunter Lindernia procumbens Philcox, Rotala indica Koehne, Elatine triandra Schkuhr)
G: Cyperus serotinus Rottboell
H: Sagittaria pygmaea Miq.

Auch für die Verbindungen (4), (6) bis (15), (19), (21), (24), (25) und (26) wurde aufgrund von ähnlichen Tests wie im vorstehenden Beispiel A bestätigt, daß sie eine ausgezeichnete selektive herbizide Wirkung und dabei keinerlei Phytotoxizität gegenüber Reispflanzen aufweisen.

Die folgenden Beispiele 1 bis 26 beschrieben die Herstellung von Verbindungen der Formel (I), und die Beispiele 27 bis 40 erläutern die Herstellung der Ausgangsverbindungen der Formel (II).

*Herstellungsbeispiele*

Beispiel 1

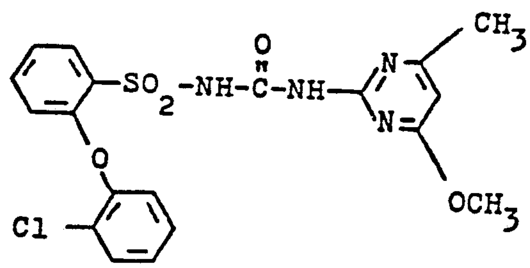

(1)

3,1 g 2-(2-Chlorophenoxy)benzolsulfonylisocyanat wurden in 13 ml Xylol gelöst. Diese Lösung wurde tropfenweise unter Rühren zu einer Suspension von 1,39 g 2-Amino-4-methoxy-6-methylpyrimidin in 13 ml Acetonitril hinzugegeben. Nach beendeter Zugabe wurde die Lösung 1 h zum Rückfluß erhitzt und dann über Nacht bei Raumtemperatur stehen gelassen. Die entstandenen Kristalle wurden abfiltriert, wodurch 4,3 g des gewünschten Produkts, N-2-(2-Chlorophenoxy)-phenylsulfonyl-N'-(4-methoxy-6-methyl-2-pyrimidyl)harnstoff erhalten wurden; Schmp. 216—220°C.

Die in der nachstehenden Tabelle 2 aufgeführten Verbindungen werden wie in Beispiel 1 beschrieben hergestellt, wobei die entsprechenden geeigneten Ausgangsstoffe verwendet werden.

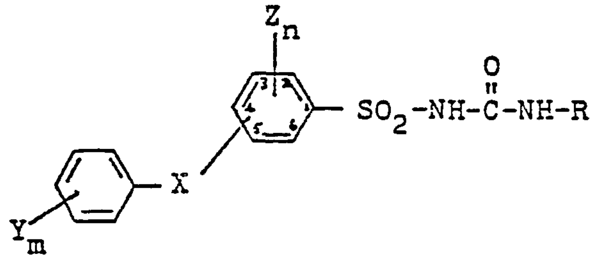

(I)

TABELLE 2

| Beispiel Nr. | 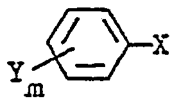 | n | Z | R | Schmelzpunkt (°C) |
|---|---|---|---|---|---|
| 2 | 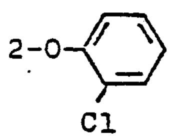 | 0 | - | 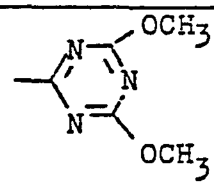 | 201-204 |
| 3 | 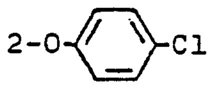 | 0 | - | 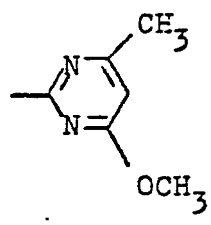 | 213-215 |
| 4 | 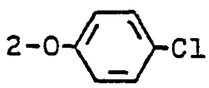 | 0 | - | 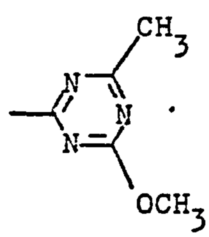 | 157-160 |

TABELLE 2 (Continued)

| Beispiel Nr. | 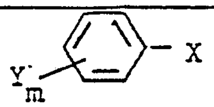 | n | Z | R | Schmelzpunkt (°C) |
|---|---|---|---|---|---|
| 5 | 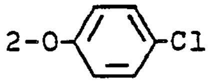 | 0 | - | 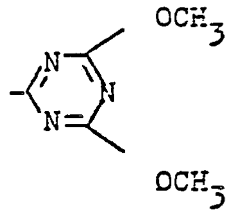 | 155-158 |
| 6 | 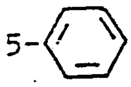 | 1 | $2-OCH_3$ | 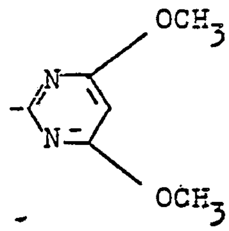 | 217-220 |
| 7 | 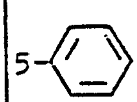 | 1 | $2-OCH_3$ | 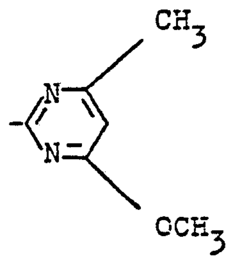 | 203-205 |
| 8 | 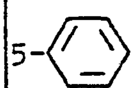 | 1 | $2-OCH_3$ | 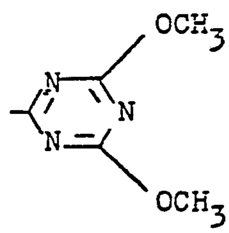 | 151-152 |
| 9 | 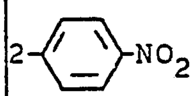 | 0 | - | 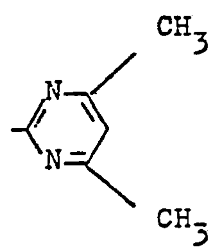 | 203-204 |

TABELLE 2 (Continued)

| Beispiel Nr. | 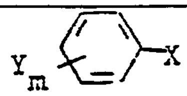 | n | Z | R | Schmelzpunkt (°C) |
|---|---|---|---|---|---|
| 10 | 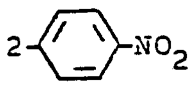 | 0 | - | 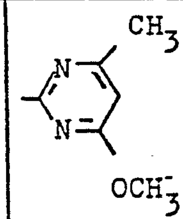 | 207-210 |
| 11 | 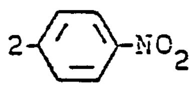 | 0 | - | 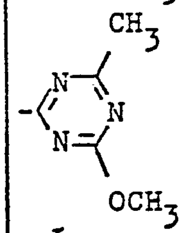 | 181-185 |
| 12 | 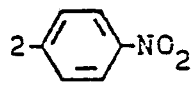 | 0 | - | 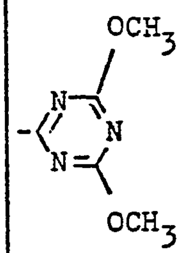 | 175-177 |
| 13 | 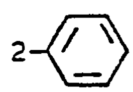 | 2 | 4-$NO_2$ 6-Br | 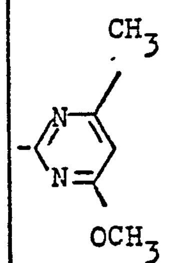 | 146-149 |
| 14 | 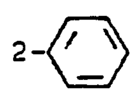 | 2 | 4-$NO_2$ 6-Br | 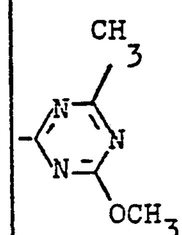 | 137-140 |
| 15 | 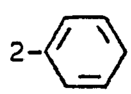 | 2 | 4-$NO_2$ 6-Br | 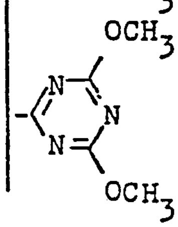 | 168-170 |

Die in der nachstehenden Tabelle 3 aufgeführten Verbindungen wurden nach der in Beispiel 1 beschriebenen Verfahrensweise unter Verwendung der angegebenen Ausgangsstoffe hergestellt.

TABELLE 3

| Beispiel Nr. | Ausgangsstoff | Ausgangsstoff | Produkt |
|---|---|---|---|
| 16 | 2-(2-Chlorophenoxy)benzol-sulfonylisocyanat | 2-Amino-4-methoxy-6-methyl-1,3,5-triazin | N-[2-(2-Chlorophenoxy)phenylsulfonyl]-N'-4-methoxy-6-methyl-1,3,5-triazin-2-yl)harnstoff |
| 17 | 2-(2-Chlorophenoxy)benzol-sulfonylisocyanat | 2-Amino-4,6-dimethoxy-pyrimidin | N-[2-(2-Chlorophenoxy)phenyl-sulfonyl]-N'-4,6-dimethoxy-2-pyrimidyl)harnstoff |
| 18 | 2-(2-Fluorophenoxy)benzol-sulfonylisocyanat | 2-Amino-4,6-dimethoxy-1,3,5-triazin | N-[2-(2-Fluorophenoxy)phenyl-sulfonyl]-N'-4,6-dimethoxy-1,3,5-triazin-2-yl)harnstoff |
| 19 | 2-(2-Fluorophenoxy)benzol-sulfonylisocyanat | 2-Amino-4-methoxy-6-methyl-1,3,5-triazin | N-[2-(2-Fluorophenoxy)phenyl-sulfonyl]-N'-4-methoxy-6-methyl-1,3,5-triazin-2-yl)harnstoff |
| 20 | 2-Chloro-6-phenoxybenzol-sulfonylisocyanat | 2-Amino-4-methoxy-6-methyl-1,3,5-triazin | N-2-Chloro-6-phenoxyphenylsulfonyl-N'-(4-methoxy-6-methyl-1,3,5-triazin-2-yl)harnstoff |
| 21 | 2-Chloro-6-phenoxybenzol-sulfonylisocyanat | 2-Amino-4,6-dimethyl-pyrimidin | N-2-Chloro-6-phenoxyphenylsulfonyl-N'-(4,6-dimethyl-2-pyrimidyl)-harnstoff |
| 22 | 2-Chloro-6-phenylbenzol-sulfonylisocyanat | 2-Amino-4,6-dimethoxy-1,3,5-triazin | N-(3-Chloro-2-biphenyl-sulfonyl)-N'-(4,6-dimethoxy-1,3,5-triazin-2-yl)harnstoff |
| 23 | 2-Chloro-6-phenylbenzol-sulfonylisocyanat | 2-Amino-4-methoxy-6-methyl-1,3,5-triazin | N-(3-Chloro-2-biphenylsulfonyl)-N'-(4-methoxy-6-methyl-1,3,5-triazin-2-yl)harnstoff |
| 24 | 5-Methyl-2-phenoxy-benzolsulfonylisocyanat | 2-Amino-4,6-dimethoxy-1,3,5-triazin | N-(5-Methyl-2-phenoxyphenyl-sulfonyl)-N'-(4,6-dimethoxy-1,3,5-triazin-2-yl)harnstoff |
| 25 | 2-(2-Methoxyphenoxy)benzol sulfonylisocyanat | 2-Amino-4-methoxy-6-methylpyrimidin | N-[2-(2-Methoxyphenoxy)phenyl-sulfonyl]-N'-(4-methoxy-6-methyl-2-pyrimidyl)harnstoff |
| 26 | 2(2,4-Dichlorophenoxy)-benzolsulfonylisocyanat | 2-Amino-4-methoxy-6-methyl 2,3,5-triazin | N-[2-(2,4-dichlorophenoxy)-phenylsulfonyl]-N'-(4-methoxy-6-methyl-1,3,5-triazin-2-yl)harnstoff |

*Herstellung der Ausgangsstoffe*

Beispiel 27

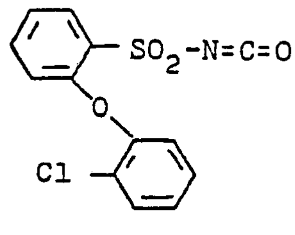

(II-1)

11,4 g 2-(2-Chlorophenoxy)benzolsulfonamid, 4 g n-Butylisocyanat und 0,5 g 1,4-Diazabicyclo[2,2,2]-octan wurden zu 50 ml Xylol gegeben, und die Mischung wurde 2 h unter Rühren zum Rückfluß erhitzt. Danach wurde die Innentemperatur auf 125°C gesenkt und eine Lösung von 6 g Trichloromethylchlorameisensäureester in 15 ml Xylol im Laufe von 2 h bei 120°C bis 125°C tropfenweise zu der vorgenannten Lösung hinzugefügt. Nach Beendigung der Zugabe wurde die Lösung 2 h erhitzt. Danach wurde die Lösung abgekühlt, unlösliche Bestandteile wurden abfiltriert, das Lösungsmittel wurde abdestilliert, und der Rückstand wurde destilliert, wonach 9,3 g des gewünschten Produkts, 2-(2-Chlorophenoxy)benzolsulfonylisocyanat erhalten wurden; Sdp. 145°C bis 148°C bei 0,67 mbar (0,5 mmHg).

Weitere substituierte Benzolsulfonylisocyanate der Formel (II), hergestellt gemäß der vorliegenden Erfindung, wie sie in der folgenden Tabelle 4 aufgeführt sind, wurden unter Verwendung der angegebenen Ausgangsstoffe entsprechend der in Beispiel 27 beschriebenen Verfahrensweise hergestellt.

TABELLE 4

| Verbind. Nr. | Ausgangsstoff | Ausgangsstoff | Produkt |
|---|---|---|---|
| 28 | 2-(4-Chlorophenoxy)benzolsulfonamid | Trichloromethylchlorameisensäureester | 2-(4-Chlorophenoxy)benzolsulfonylisocyanat |
| 29 | 2-(2-Fluorophenoxy)benzolsulfonamid | Phosgen | 2-(2-Fluorophenoxy)benzolsulfonylisocyanat |
| 30 | 2-Chloro-6-phenoxybenzolsulfonamid | Trichloromethylchlorameisensäureester | 2-Chloro-6-phenoxybenzolsulfonylisocyanat |
| 31 | 2-Chloro-6-phenylbenzol sulfonamid | Trichloromethylchlor ameisensäureester | 2-Chloro-6-phenylbenzolsulfonylisocyanat |
| 32 | 2-Methoxy-5-phenylbenzolsulfonamid | Phosgen | 2-Methoxy-5-phenylbenzolsulfonylisocyanat |
| 33 | 2-Ethoxy-5-phenylbenzolsulfonamid | Phosgen | 2-Ethoxy-5-phenylbenzolsulfonylisocyanat |
| 34 | 2-(2-Methoxyphenoxy)-benzolsulfonamid | Phosgen | 2-(2-Methoxyphenoxy)benzolsulfonylisocyanat |
| 35 | 2-(4-Nitrophenyl)benzolsulfonamid | Trichloromethylchlorameisensäureester | 2-(4-Nitrophenyl)benzolsulfonylisocyanat |
| 36 | 2-(o-Tolyloxy)benzolsulfonamid | Phosgen | 2-(o-Tolyloxy)benzolsulfonylisocyanat |
| 37 | 5-Methyl-2-phenoxybenzolsulfonamid | Phosgen | 5-Methyl-2-phenoxybenzolsulfonylisocyanat |
| 38 | 2-(4-Isopropylphenoxy)-benzolsulfonamid | Trichloromethylchlorameisensäureester | 2-(4-Isopropylphenoxy)benzolsulfonylisocyanat |
| 39 | 2-Bromo-4-nitro-6-phenyl-benzolsulfonamid | Trichloromethylchlorameisensäureester | 2-Bromo-4-nitro-6-phenylbenzolsulfonylisocyanat |
| 40 | 2-(2,4-Dichlorophenoxy)-benzolsulfonamid | Phosgen | 2-(2,4-Dichlorophenoxy)benzolsulfonylisocyanat |

**Patentansprüche für den Vertragsstaat: AT**

1. Verfahren zur Herstellung von substituierten Phenylsulfonylharnstoffderivaten der allgemeinen Formel I

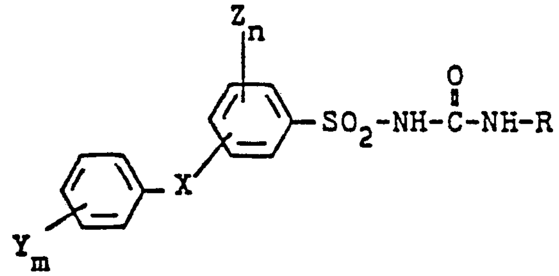

(I)

in der

X für ein Sauerstoff-Atom oder eine Einfachbindung steht,

Y und Z unabhängig voneinander ein Halogen-Atom, eine $C_1$- bis $C_6$-Alkyl-Gruppe, eine $C_1$- bis $C_6$-Alkoxy-Gruppe oder eine Nitro-Gruppe und

R eine Gruppe der allgemeinen Formel

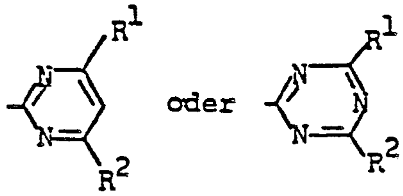

bezeichnen, in der

$R^1$ und $R^2$ unabhängig voneinander für eine $C_1$- bis $C_6$-Alkyl-Gruppe oder eine $C_1$-bis $C_6$-Alkoxy-Gruppe stehen, und

m und n jeweils unabhängig voneinander 0, 1 oder 2 sind, mit der Maßgabe, daß m und n nicht beide gleichzeitig O sind und daß Z — wenn n = 2 ist — unterschiedliche Substituenten in form von Br und $NO_2$ bedeuten kann, dadurch gekennzeichnet, daß eine Verbindung der allgemeinen Formel II

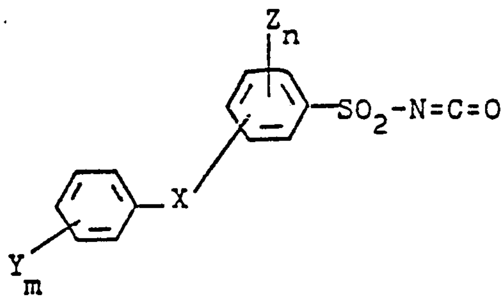

(II)

in der X, Y, Z, m und n die vorstehend angegebenen Bedeutungen haben, mit einer Verbindung der allgemeinen Formel III

$$H_2N—R \quad (III),$$

in der R die vorstehend angegebene Bedeutung hat, umgesetzt wird, gegebenenfalls in Geganwart eines inerten organischen Lösungsmittels oder Verdünnungsmittels und/oder in Gegenwart eines Katalysators.

2. Verfahren zur Herstellung von substituierten Benzolsulfonylisocyanaten der allgemeinen Formel II

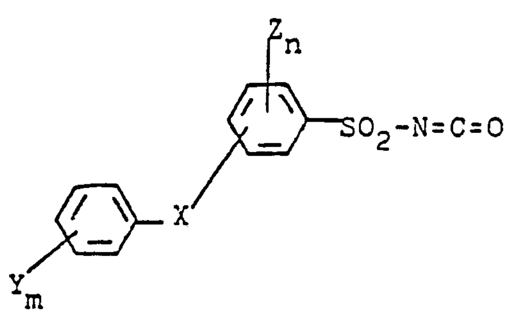

(II)

in der

X für ein Sauerstoff-Atom oder eine Einfachbindung steht,

Y und Z unabhängig voneinander ein Halogen-Atom, eine $C_1$- bis $C_6$-Alkyl-Gruppe, eine $C_1$- bis $C_6$-Alkoxy-Gruppe oder eine Nitro-Gruppe bezeichnen und

m und n jeweils unabhängig voneinander 0, 1 oder 2 sind, mit der Maßgabe, daß m und n nicht beide gleichzeitig 0 sind und daß Z — wenn n = 2 ist — unterschiedliche Substituenten in Form von Br und $NO_2$ bedeuten kann,

dadurch gekennzeichnet, daß substituierte Benzolsulfonamide der allgemeinen Formel IV

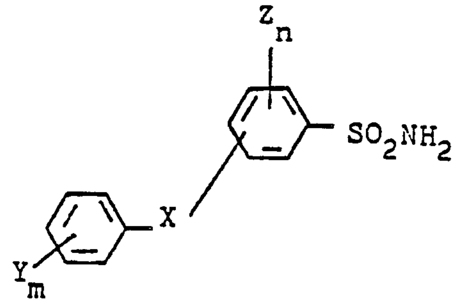

(IV)

in der

X, Y, Z, m und n die vorstehend angegebenen Bedeutungen haben, mit Phosgen ($COCl_2$) oder Trichloromethylchlorameisensäureester ($CCl_3OCOCl$), in Gegenwart eines Kohlenwasserstoff-Isocyanats, gegebenenfalls in Gegenwart eines inerten organischen Lösungsmittels oder Verdünnungsmittels und/oder in Gegenwart eines tertiären Amins als Katalysator, umgesetzt werden.

3. Herbizide Mittel, gekennzeichnet durch einen Gehalt an mindestens einem substituierten Phenylsulfonylharnstoff-Derivat der Formel (I) gemäß Anspruch 1.

4. Verwendung von substituierten Phenylsulfonylharnstoff-Derivaten der Formel (I) gemäß Anspruch 1 zur Bekämpfung von unerwünschtem Pflanzenwachstum.

5. Verfahren zur Herstellung von herbiziden Mitteln, dadurch gekennzeichnet, daß man substituierte Phenylsulfonylharnstoff-Derivate der Formel (I) gemäß Anspruch 1 mit Streckmitteln und/oder oberflächenaktiven Mitteln vermischt.

**Revendications pour l'Etat contractant: AT**

1. Procédé de production de dérivés substitués de phénylsulfonylurée de formule générale I

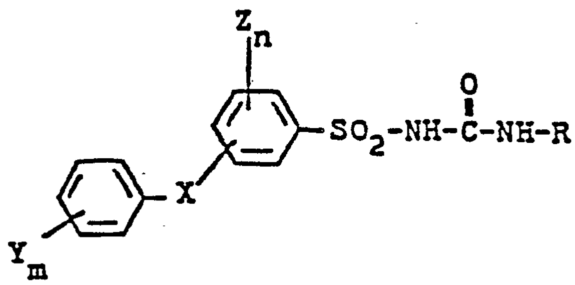

(I)

dans laquelle

X représente un atome d'oxygène ou une liaison simple,

Y et Z représentent, indépendamment l'un de l'autre, un atome d'halogène, un groupe alkyle en $C_1$ à $C_6$, un groupe alkoxy en $C_1$ à $C_6$ ou un groupe nitro et

R est un groupe de formule générale

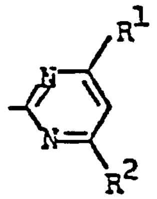

ou

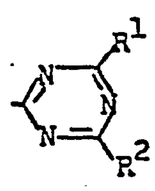

dans laquelle

$R^1$ et $R^2$ représentent, indépendamment l'un de l'autre, un groupe alkyle en $C_1$ à $C_6$ ou un groupe alkoxy en $C_1$ à $C_6$, et

*m* et *n* ont chacun, indépendamment l'un de l'autre, la valeur 0, 1 ou 2, sous réserve que *m* et *n* ne soient pas tous deux en même temps égaux à O et que Z, lorsque *n* est égal à 2, puisse représenter des substituants différents sous la forme de Br et $NO_2$,

caractérisé en ce qu'on fait réagir un composé de formule générale II

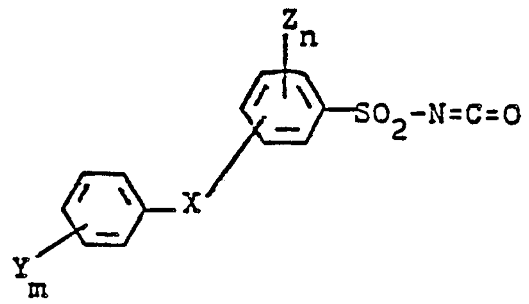

(II)

dans laquelle X, Y, Z, *m* et *n* ont les définitions indiquées ci-dessus, avec un composé de formule générale III

$H_2N—R$ (III),

dans laquelle R a la définition indiquée ci-dessus, éventuellement et présence d'un solvant ou diluant organique inerte et/ou en présence d'un catalyseur.

2. Procédé de production de benzènesulfonylisocyanates substitués de formule générale II

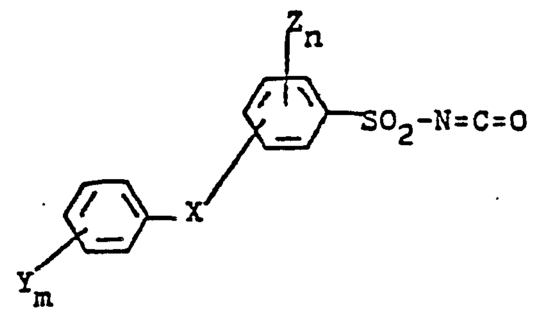

(II)

dans laquelle

X désigne un atome d'oxygène ou une liaison simple,

Y et Z représentent, indépendamment l'un de l'autre, un atome d'halogène, un groupe alkyle en $C_1$ à $C_6$, un groupe alkoxy en $C_1$ à $C_6$ ou un groupe nitro et

*m* et *n* ont chacun, indépendamment l'un de l'autre, la valeur 0, 1 ou 2, sous réserve que *m* et *n* ne soient pas tous deux en même temps égaux à 0 et que Z — lorsque *n* est égal à 2 — puisse désigner des substituants différents sous la forme de Br et $NO_2$,

caractérisé en ce qu'on fait réagir des benzènesulfonamides substitués de formule générale IV

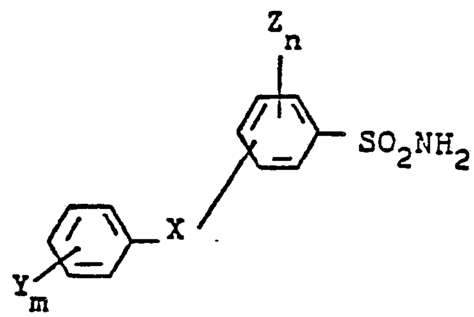

(IV)

dans laquelle

X, Y, Z, *m* et *n* ont les définitions indiquées ci-dessus, avec le phosgène ($COCl_2$) ou l'ester trichlorométhylique de l'acide chloroformique ($CCl_3OCOCl$) en présence d'un isocyanate d'hydrocarbure, éventuellement en présence d'un solvant ou diluant organique inerte et/ou en présence d'une amine tertiaire comme catalyseur.

3. Compositions herbicides, caractérisées par une teneur en au moins un dérivé substitué de phénylsulfonylurée de formule (I) suivant la revendication 1.

4. Utilisation de dérivés substitués de phénylsulfonylurée de formule (I) suivant la revendication 1 pour combattre une végétation indésirable.

5. Procédé de préparation de compositions herbicides, caractérisé en ce qu'on mélange des dérivés substitués de phénylsulfonylurée de formule (I) suivant la revendication 1 avec des diluants et/ou des agents tensio-actifs.

**Claims for the Contracting State: AT**

1. Process for the preparation of substituted phenylsulphonylurea derivatives of the general formula I

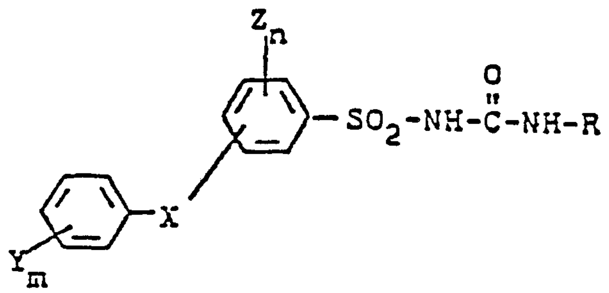

(I)

in which

X represents an oxygen atom or a single bond,

Y and Z independently of one another designate a halogen atom, a $C_1$ to $C_6$ alkyl group, a $C_1$ to $C_6$ alkoxy group or a nitro group and

R designates a group of the general formula

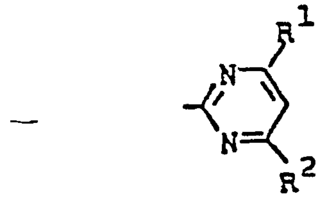

or

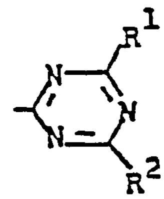

in which

$R^1$ and $R^2$ independently of one another represent a $C_1$ to $C_6$ alkyl group or a $C_1$ to $C_6$ alkoxy group and

m and n each independently of one another are 0, 1 or 2, with the proviso that m and n are not both O at the same time and that Z can denote different substituents in the form of Br and $NO_2$ when n = 2, characterised in that a compound of the general formula II

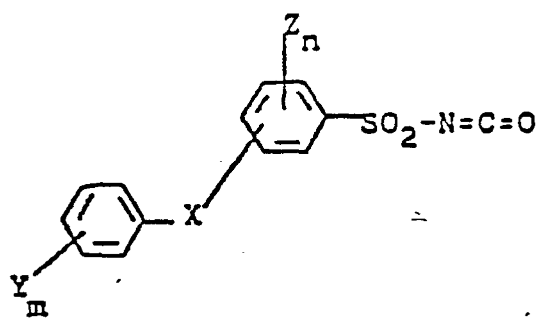

in which

X, Y, Z, m and n have the aforementioned meanings, is reacted with a compound of the general formula III

$$H_2N—R \qquad (III),$$

in which

R has the aforementioned meaning,

if appropriate in the presence of an inert organic solvent or diluent and/or in the presence of a catalyst.

2. Process for the preparation of substituted benzenesulphonyl isocyanates of the general formula II

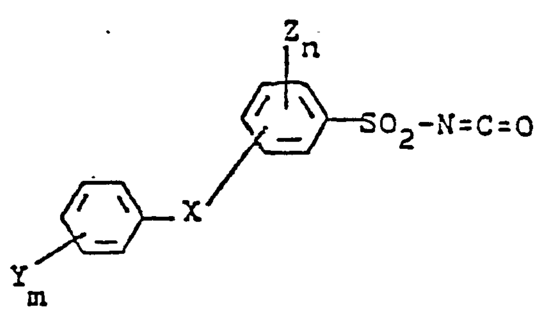

(II)

in which

X represents an oxygen atom or a single bond,

Y and Z independently of one another designate a halogen atom, a $C_1$ to $C_6$ alkyl group, a $C_1$ to $C_6$ alkoxy group or a nitro group and

m and n each independently of one another are 0, 1 or 2, with the proviso that m and n are not both 0 at the same time and that Z can denote different substituents in the form of Br and $NO_2$ when n = 2, characterised in that substituted benzenesulphonamides of the general formula IV

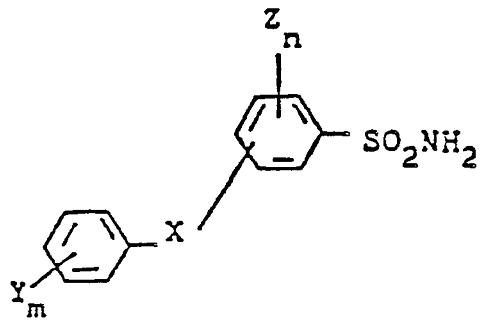

(IV)

in which

X, Y, Z, m and n have the aforementioned meanings, are reacted with phosgene ($COCl_2$) or trichloromethyl chloroformic acid ester ($CCl_3OCOCl$), in the presence of a hydrocarbon isocyanate, if appropriate in the presence of an inert organic solvent or diluent and/or in the presence of a tertiary amine as the catalyst.

3. Herbicidal agents, characterised in that they contain at least one substituted phenylsulphonylurea derivative of the formula (I) according to Claim 1.

4. Use of substituted phenylsulphonylurea derivatives of the formula (I) according to Claim 1 for combating undesired plant growth.

5. Process for the preparation of herbicidal agents, characterised in that substituted phenylsulphonylurea derivatives of the formula (I) according to Claim 1 are mixed with extenders and/or surface-active agents.